# EUROPEAN PATENT APPLICATION

(11) **EP 4 324 848 A1**
(43) Date of publication of application: **21.02.2024**
(21) Application number: 22788458.2
(22) Date of filing: 13.04.2022
(51) Int. Cl.: C07K 16/10, A61P 31/14, G01N 33/569, A61K 39/00

(54) **HUMAN ANTIBODY TARGETING COVID-19 VIRUS**

(30) Priority: 16.04.2021 KR 20210049528; 01.12.2021 KR 20210169632
(71) Applicant: Korea University Research and Business Foundation, Seoul 02841 (KR)
(72) Inventor: JUNG, Sang Taek, Seongnam-si, Gyeonggi-do 13562 (KR); LEE, Ji Sun, Seoul 03472 (KR); KIM, Bo Mi, Suwon-si, Gyeonggi-do 16316 (KR)
(74) Representative: Isarpatent
(86) International application number: PCT/KR2022/005382
(87) International publication number: WO 2022/220603

(57) **Abstract**

The present invention relates to a human antibody against a novel coronavirus and a variant thereof, or an immunologically active fragment of the human antibody. The human antibody against a novel coronavirus and a variant thereof, or an immunologically active fragment of the human antibody can effectively keep COVID-19 virus from infecting a host by binding with high affinity to an RBD region of a spike protein of COVID-19 virus or a variant thereof, may be utilized for preventing or treating COVID-19 virus infection by binding to a variety of all variants of COVID-19 virus with high affinity, and may be advantageously utilized in diagnosis and epidemiological surveys of highly infectious covid-19 virus by enabling rapid diagnosis (detection) of various types of COVID-19 virus.

## Description

### [Technical Field]

The present invention relates to a human antibody against a novel coronavirus (COVID-19 virus: SARS-CoV-2) and a variant thereof, or an immunologically active fragment of the human antibody.

### [Background Art]

Coronaviruses are enveloped viruses having single-stranded positive RNA genomes and have been isolated from a variety of animals including humans since they were first found in 1937. Coronaviruses may be divided into four groups. Among them, alpha-coronavirus and beta-coronavirus mainly infect mammals, while gamma-coronavirus and delta-coronavirus infect birds. However, recently, there has been a confirmed case of infection in pigs with delta-coronavirus. Coronaviruses may be propagated heterogeneously. Representative examples include the SARS coronavirus that caused severe acute respiratory syndrome that was prevalent worldwide in 2003 and the MERS coronavirus that spread in Korea in 2015, both of which are known to be originated from bats. Among mammals, bats have the ability to fly, so they may inhabit a wide range of areas. Due to the nature of many subjects living in groups in small spaces such as caves, when one subject is infected with a virus, the infection may be transmitted in groups and spread to other animals through movement over a wide area through flight. In addition, unlike other mammals, bats have a high body temperature and are resistant to viruses, so bats, which are coronavirus hosts, are not affected and may continue to spread infection.

As described above, coronaviruses may be propagated heterogeneously, and novel coronaviruses originated from bats may spread to humans and cause great problems. Therefore, there is an urgent need for development of effective diagnostic methods and therapeutic agents to detect novel bat-originated coronaviruses. Currently, research on antiviral agents is actively underway in Korea and other countries. Non-specific antiviral agents that have shown efficacy in clinical practice include type I interferon and ribavirin but have been reported to have antiviral effects only in the early stages of infection. In response to the increasing need for development, therapeutic agents for MERS-CoV and SARS-CoV have recently attracted attention (A. Zumla et.al, Nature Reviews Drug Discovery, 15:327-347,2016). However, there are currently neither approved therapeutic agents nor vaccines in both Korea and other countries, and a system for evaluating developed substances has not been established. Therefore, there is an urgent need for development of a system for evaluating the efficacy of the substances.

COVID-19 virus infection (novel coronavirus infection, COVID-19), a novel coronavirus reported to have occurred in Wuhan, China in December 2019, has an incubation period of approximately 2 to 14 days after infection, followed by main symptoms of fever, respiratory symptoms such as coughing or difficulty breathing, and pneumonia. Cases of asymptomatic infection are rare but are occurring. The World Health Organization (WHO) declared COVID-19 to be a pandemic, the highest alert level. As of 21:00 on April 11, 2020, COVID-19 is a disease with a fatality rate of 6.05%, with 1,715,224 confirmed cases and 103,827 deaths in 215 countries around the world. The genome of the COVID-19 virus is an RNA virus, with an envelope surrounding the protein capsid containing the genome, and spike proteins in the form of membrane glycoproteins protrude like protrusions on the surface. Among them, the spike protein plays an important role in infecting the host with a virus by binding to the surface of a receptor present on the host that protrudes like a spike and infiltrating into the host cell. The spike protein is functionally divided into an S1 subunit that binds to the receptor of the host cell and an S2 subunit that fuses to the membrane. The S1 subunit has an RBD (receptor binding domain) that binds to the ACE2 (Angiotensin converting enzyme 2) receptor of the host cell. The RBD region of the spike protein is very suitable as a target for the development of human antibodies with virus neutralizing ability to prevent viruses from infecting host cells. In fact, the human antibodies with the ability to neutralize the SARS epidemic in 2003 and the MERS epidemic in 2015 announced in previous research were all antibodies that bound to the RBD region of the coronavirus. However, various mutations have occurred in the spike protein of the COVID-19 virus, causing various variants to break through and infect, so countermeasures are needed.

Accordingly, a novel human antibody with virus-neutralizing ability produced by binding to the RBD region of the COVID-19 virus or its variants using an ultra-fast search system was discovered.

### [Disclosure]

### [Technical Problem]

An aspect of the present invention is directed to providing an antibody or an immunologically active fragment for use in the detection of COVID-19 virus or a variant thereof, prevention or treatment of COVID-19 virus infection.

### [Technical Solution]

An embodiment of the present invention provides an antibody specific to COVID-19 virus or a variant thereof, or an immunologically active fragment thereof.

In addition, an embodiment of the present invention provides an isolated nucleic acid molecule encoding the antibody or the immunologically active fragment thereof, a vector containing the same, and a host cell transformed with the vector.

In addition, an embodiment of the present invention provides a method of producing an antibody specific to COVID-19 virus or a variant thereof, or an immunologically active fragment thereof.

In addition, an embodiment of the present invention provides a pharmaceutical composition for preventing or treating COVID-19 virus infection.

In addition, an embodiment of the present invention provides a composition for detecting COVID-19 virus or a variant thereof.

In addition, an embodiment of the present invention provides a kit for detecting COVID-19 virus or a variant thereof.

In addition, an embodiment of the present invention provides a method for detecting COVID-19 virus or a variant thereof.

In addition, an embodiment of the present invention provides a method of providing information regarding diagnosis of COVID-19 virus infection.

In addition, an embodiment of the present invention provides a use of an antibody specific to COVID-19 virus (SARS-CoV-2) or a variant thereof, or an immunologically active fragment thereof, for use in preparing a therapeutic agent for COVID-19 virus infection.

In addition, an embodiment of the present invention provides a use of an antibody specific to COVID-19 virus (SARS-CoV-2) or a variant thereof, or an immunologically active fragment thereof, for preventing or treating COVID-19 virus infection.

In addition, an embodiment of the present invention provides a method of treating COVID-19 virus infection, in which the method includes administering a pharmaceutically effective amount of an antibody specific to COVID-19 virus (SARS-CoV-2) or a variant thereof, or an immunologically active fragment thereof, to a subject suffering from the COVID-19 virus infection.

### [Advantageous Effects]

A human antibody and an immunologically active fragment thereof that bind to a RBD region of COVID-19 virus or a variant thereof, which are causative agents of COVID-19 disease, according to an embodiment of the present invention can effectively keep COVID-19 virus from infecting a host by binding with high affinity to an RBD region of a spike protein of COVID-19 virus or a variant thereof, may be utilized for preventing or treating COVID-19 virus infection by binding to all of a COVID-19 virus (SARS-CoV-2) wild type, a COVID-19 virus alpha variant, a COVID-19 virus beta variant, a COVID-19 virus gamma variant, a COVID-19 virus delta variant, a COVID-19 virus kappa variant, and a COVID-19 virus mu variant with high affinity, and may be advantageously utilized in diagnosis and epidemiological surveys of highly infectious COVID-19 virus by enabling rapid immunodiagnosis (detection) of various types of COVID-19 virus using the antibody and the immunologically active fragments thereof.

### [Description of Drawings]

FIG. 1 is a diagram illustrating the expression and purification of a dimeric COVID-19 virus-RBD region antigen protein expression vector and a tetrameric COVID-19 virus-RBD region antigen protein expression vector produced in an embodiment of the present invention in animal cells and the identification by SDS-PAGE gel.
FIG. 2 is a schematic diagram illustrating antibody search using a flow cytometer.
FIG. 3 is a diagram illustrating an iterative screening process to amplify human antibody clones showing specific binding to a COVID-19 virus-RBD region.
FIG. 4 is a diagram illustrating the amino acid sequences of six scFv human antibody variants showing high binding affinity to the COVID-19 virus-RBD region.
FIG. 5 is a diagram illustrating the amino acid sequences of four VH human antibody variants showing high binding affinity to the COVID-19 virus-RBD region.
FIG. 6 is a diagram illustrating the results of analysis of COVID-19 virus-RBD binding activity of six types of scFv antibody variants through flow cytometry.
FIG. 7 is a diagram illustrating the results of analysis of COVID-19 virus-RBD binding activity of four types of VH antibody variants through flow cytometry.
FIG. 8 is a diagram illustrating the expression and purification of IgG-type antibodies of scFv variants JS 1, JS 2, JS 3, JS 4, JS 5, and JS 7 and the identification by SDS-PAGE gel.
FIG. 9 is a diagram identifying the binding activity of IgG-type antibodies of scFv variants JS 1, JS 2, JS 3, and JS 4 by ELISA.
FIG. 10 is a diagram illustrating an expression vector for producing an IgG-type antibody produced by introducing VH variant JS-VH 4 as a dimer/tetramer (top), and its expression and purification, and the identification by SDS-PAGE gel (bottom).
FIG. 11 is a diagram identifying the binding activity of an IgG-type antibody produced by introducing the VH variant JS-VH 4 as a dimer/tetramer by ELISA.
FIG. 12 is a diagram illustrating the results of analysis of COVID-19 virus-RBD binding activity of five types of scFv variants originated from JS-VH 4.
FIG. 13 is a diagram illustrating the results of amino acid sequence analysis of five types of scFv antibody variants originated from JS-VH 4.
FIG. 14 is a diagram comparing the binding activity to (BM 13-1, BM 18-1, BM 57-1, and BM 73-1) COVID-19 virus-RBD by substituting four types (BM 13, BM 18, BM 57, and BM 73) of amino acid mutations occurred in a VH region among the antibody variants originated from JS-VH 4 with the sequence of JS-VH 4 again.
FIG. 15 is a diagram illustrating the expression vector for the RBD antigen protein of the SARS-CoV-2 virus delta variant, and the results of SDS-PAGE analysis of size and purity after expression and purification.
FIG. 16 is a schematic diagram illustrating the production of a yeast display library for selecting variants with improved binding activity to the RBD antigen protein of the SARS-CoV-2 virus delta variant.
FIG. 17 is a diagram illustrating the results of FACS analysis of sub-libraries of each round screened through yeast cell wall display.
FIG. 18 is a diagram illustrating the amino acid sequence of Fab antibody JS18.1, which shows high binding affinity to the RBD of the SARS-CoV-2 virus delta variant.
FIG. 19 is a diagram illustrating heavy and light chain expression vectors for expressing IgG-type antibodies of Fab antibody JS18.1, which shows high binding affinity to the RBD of the SARS-CoV-2 virus delta variant, and expression and purification thereof in animal cells and identification thereof by SDS-PAGE gel.
FIG. 20 is a diagram illustrating the analysis result by SDS-PAGE after expression and purification of the RBD of a wild type (WT), an alpha variant, a beta variant, a gamma variant, a delta variant, a kappa mutant, and a mu variant of the SARS-CoV-2 virus as an antigen protein in the form of a monomer.
FIG. 21 is a diagram illustrating the results of analyzing the binding activity to a wild type (WT), an alpha variant, a beta variant, a gamma variant, a delta variant, a kappa mutant, and a mu variant of the SARS-CoV-2 virus of a JS18.1 antibody and its parent antibody, BM-18 antibody.

### [Modes of the Invention]

Hereinafter, the present invention will be described in detail by way of embodiments of the present invention with reference to the accompanying drawings. However, the following examples are provided by way of illustration of the present invention. When it is determined that the specific description of known techniques or configuration well known to those skilled in the art unnecessarily obscure the gist of the present invention, the detailed description therefor may be omitted, and the present invention is not limited thereto. The present invention allows various modifications and applications within the description of the claims to be described later and the scope of equivalents interpreted therefrom.

Further, terminologies used herein are terms used to properly represent preferred embodiments of the present invention. It may vary depending on the intent of users or operators, or custom in the art to which the present invention belongs. Accordingly, the definitions of these terms should be based on the contents throughout this specification. In the entire specification, when a part is referred to as "comprising" a component, it means that it may further include other components without excluding other components unless specifically described otherwise.

Unless otherwise defined, all the technical terms used herein are used with the same meaning as commonly understood by a person skilled in the art in the field related to the present invention. In addition, preferred methods or samples are described herein, but those similar or equivalent thereto are incorporated in the scope of the present invention. The contents of all the publications disclosed as references herein are incorporated in the present invention.

Throughout the specification, the conventional one-letter and three-letter codes for amino acids present in nature are used, as well as generally accepted three-letter codes for other amino acids, such as Alb (α-aminoisobutyric acid) and Sar (N-methylglycine). Additionally, the amino acids mentioned in abbreviation in the present invention are described according to the IUPAC-IUB Nomenclature below.

Alanine: A, arginine: R, asparagine: N, aspartic acid: D, cysteine: C, glutamic acid: E, glutamine: Q, glycine: G, histidine: H, isoleucine: I, leucine: L, lysine: K, methionine: M, phenylalanine: F, proline: P, serine: S, threonine: T, tryptophan: W, tyrosine: Y, and valine: V.

In an aspect, an embodiment of the present invention relates to an antibody specific to COVID-19 virus (SARS-CoV-2) or a variant thereof, or an immunologically active fragment thereof.

In an embodiment, it may be an FC variant or Fab variant of an antibody specific to COVID-19 virus or a variant thereof.

In an embodiment, the variant may be an alpha variant, a beta variant, a gamma variant, a delta variant, a kappa variant, or a mu variant.

In an embodiment, the immunologically active fragment may be any one selected from the group consisting of Fab, Fd, Fab', dAb, F(ab'), F(ab')₂, single chain fragment variable (scFv), Fv, single chain antibody, Fv dimer, complementarity determining region fragment, humanized antibody, a chimeric antibody and a diabody, and may more preferably be scFv or Fab.

In an embodiment, the antibody specific to COVID-19 virus (SARS-CoV-2) or the variant thereof, or the immunologically active fragment thereof of an embodiment of the present invention may include a VH domain including Complementarity Determining Regions Heavy Chain (CDRH)1, which includes any one selected from the group consisting of amino acid sequences represented by SEQ ID NOs: 1 to 8, CDRH2, which includes any one selected from the group consisting of amino acid sequences represented by SEQ ID NOs: 9 to 14, and CDRH3, which includes any one selected from the group consisting of amino acid sequences represented by SEQ ID NOs: 15 to 23.

In an embodiment, the antibody specific to COVID-19 virus (SARS-CoV-2) or the variant thereof, or the immunologically active fragment thereof of an embodiment of the present invention may include a VH domain including FR1, which includes any one selected from the group consisting of amino acid sequences represented by SEQ ID NOs: 47 to 50, FR2, which includes any one selected from the group consisting of amino acid sequences represented by SEQ ID NOs: 51 to 56, FR3, which includes any one selected from the group consisting of amino acid sequences represented by SEQ ID NOs: 57 to 61, and FR4, which includes an amino acid sequence represented by SEQ ID NO: 62 or 65.

In an embodiment, the antibody specific to COVID-19 virus (SARS-CoV-2) or the variant thereof, or the immunologically active fragment thereof of an embodiment of the present invention may include complementarity determining regions light chain (CDRL) 1, which includes any one selected from the group consisting of amino acid sequences represented by SEQ ID NOs: 24 to 32, CDRL2, which includes any one selected from the group consisting of amino acid sequences represented by SEQ ID NOs: 33 to 37, and CDRL3, which includes any one selected from the group consisting of amino acid sequences represented by SEQ ID NOs: 38 to 46.

In an embodiment, the antibody specific to COVID-19 virus (SARS-CoV-2) or the variant thereof, or the immunologically active fragment thereof of an embodiment of the present invention may include a VL domain including FR1, which includes any one selected from the group consisting of amino acid sequences represented by SEQ ID NOs: 66 to 75, FR2, which includes any one selected from the group consisting of amino acid sequences represented by SEQ ID NOs: 76 to 85, FR3, which includes any one selected from the group consisting of amino acid sequences represented by SEQ ID NOs: 86 to 94, and FR4, which includes any one selected from the group consisting of amino acid sequences represented by SEQ ID NO: 95 to 103.

In an embodiment, the antibody specific to COVID-19 virus (SARS-CoV-2) or the variant thereof, or the immunologically active fragment thereof of an embodiment of the present invention may include a VH domain including FR1, which includes any one selected from the group consisting of amino acid sequences represented by SEQ ID NOs: 47 to 50, FR2, which includes any one selected from the group consisting of amino acid sequences represented by SEQ ID NOs: 51 to 56, FR3, which includes any one selected from the group consisting of amino acid sequences represented by SEQ ID NOs: 57 to 61, and FR4, which includes an amino acid sequence represented by SEQ ID NO: 62 or 65.

In an embodiment, the antibody specific to COVID-19 virus (SARS-CoV-2) or the variant thereof, or the immunologically active fragment thereof of an embodiment of the present invention may include a VL domain including Complementarity Determining Regions Light Chain (CDRL)1, which includes any one selected from the group consisting of amino acid sequences represented by SEQ ID NOs: 24 to 32, CDRL2, which includes any one selected from the group consisting of amino acid sequences represented by SEQ ID NOs: 33 to 37, and CDRL3, which includes any one selected from the group consisting of amino acid sequences represented by SEQ ID NOs: 38 to 46.

In an embodiment, the antibody specific to COVID-19 virus (SARS-CoV-2) or the variant thereof, or the immunologically active fragment thereof of an embodiment of the present invention may include a VL domain including FR1, which includes any one selected from the group consisting of amino acid sequences represented by SEQ ID NOs: 66 to 75, FR2, which includes any one selected from the group consisting of amino acid sequences represented by SEQ ID NOs: 76 to 85, FR3, which includes any one selected from the group consisting of amino acid sequences represented by SEQ ID NOs: 86 to 94, and FR4, which includes any one selected from the group consisting of amino acid sequences represented by SEQ ID NOs: 95 to 103.

In an embodiment, the antibody specific to COVID-19 virus (SARS-CoV-2) or the variant thereof, or the immunologically active fragment thereof of an embodiment of the present invention may include a V domain including (i) a VH domain including CDRH1, which includes any one selected from the group consisting of amino acid sequences represented by SEQ ID NOs: 1 to 8, CDRH2, which includes any one selected from the group consisting of amino acid sequences represented by SEQ ID NOs: 9 to 14, and CDRH3, which includes any one selected from the group consisting of amino acid sequences represented by SEQ ID NOs: 15 to 23; and/or (ii) a VL domain including CDRL1, which includes any one selected from the group consisting of amino acid sequences represented by SEQ ID NOs: 24 to 32, CDRL2, which includes any one selected from the group consisting of amino acid sequences represented by SEQ ID NOs: 33 to 37, and CDRL3, which includes any one selected from the group consisting of amino acid sequences represented by SEQ ID NOs: 38 to 46.

In an embodiment, the antibody specific to COVID-19 virus (SARS-CoV-2) or the variant thereof, or the immunologically active fragment thereof of an embodiment of the present invention may include a VH domain, which includes any one selected from the group consisting of amino acid sequences represented by SEQ ID NOs: 104 to 119; and a VL domain, which includes any one selected from the group consisting of amino acid sequences represented by SEQ ID NOs: 120 to 131.

In an embodiment, the antibody specific to COVID-19 virus (SARS-CoV-2) or the variant thereof, or the immunologically active fragment thereof of an embodiment of the present invention may include a V domain including i) a VH domain including FR1, which includes any one selected from the group consisting of amino acid sequences represented by SEQ ID NOs: 47 to 50, CDRH1, which includes any one selected from the group consisting of amino acid sequences represented by SEQ ID NOs: 1 to 8, FR2, which includes any one selected from the group consisting of amino acid sequences represented by SEQ ID NOs: 51 to 56, CDRH2, which includes any one selected from the group consisting of amino acid sequences represented by SEQ ID NOs: 9 to 14, FR3, which includes any one selected from the group consisting of amino acid sequences represented by SEQ ID NOs: 57 to 61, CDRH3, which includes any one selected from the group consisting of amino acid sequences represented by SEQ ID NOs: 15 to 23, and FR4, which includes any one selected from the group consisting of an amino acid sequence represented by SEQ ID NO: 62 or 65; and/or ii) a VL domain including FR1, which includes any one selected from the group consisting of amino acid sequences represented by SEQ ID NOs: 66 to 75, CDRL1, which includes any one selected from the group consisting of amino acid sequences represented by SEQ ID NOs: 24 to 32, FR2, which includes any one selected from the group consisting of amino acid sequences represented by SEQ ID NOs: 76 to 85, CDRL2, which includes any one selected from the group consisting of amino acid sequences represented by SEQ ID NOs: 33 to 37, FR3, which includes any one selected from the group consisting of amino acid sequences represented by SEQ ID NOs: 86 to 94, CDRL3, which includes any one selected from the group consisting of amino acid sequences represented by SEQ ID NOs: 38 to 46, and FR4, which includes any one selected from the group consisting of amino acid sequences represented by SEQ ID NO: 95 to 103.

In an embodiment, the antibody specific to COVID-19 virus or the immunologically active fragment thereof of an embodiment of the present invention may be any one variant selected from the group consisting of scFv variants of JS 1, JS 2, JS 3, JS 4, JS 5, and JS 7; VH variants of JS-VH 4, JS-VH 9, JS-VH 19, and JS-VH 21; JS-VH 4-originated scFv variants of BM 13, BM 18, BM 57, and BM 73; and a BM 18-originated scFv variant of JS18.1.

In an embodiment, the antibody specific to COVID-19 virus (SARS-CoV-2) or the variant thereof, or the immunologically active fragment thereof of an embodiment of the present invention may include CL sequence including an amino acid sequence represented by SEQ ID NO: 132, CH1 sequence including an amino acid sequence represented by SEQ ID NO: 133, CH2 sequence including an amino acid sequence represented by SEQ ID NO: 134, or CH3 sequence including an amino acid sequence represented by SEQ ID NO: 135.

In an embodiment, the antibody specific to COVID-19 virus (SARS-CoV-2) or the variant thereof, or the immunologically active fragment thereof of an embodiment of the present invention may be produced to include a dimer or tetramer when produced as an IgG-type antibody.

In an embodiment, the antibody specific to COVID-19 virus (SARS-CoV-2) or the variant thereof, or the immunologically active fragment thereof of an embodiment of the present invention may specifically bind to any one receptor binding domain (RBD) selected from the group consisting of a COVID-19 virus (SARS-CoV-2) wild type, a COVID-19 virus alpha variant, a COVID-19 virus beta variant, a COVID-19 virus gamma variant, a COVID-19 virus delta variant, a COVID-19 virus kappa variant, and a COVID-19 virus mu variant, in which the RBD of the COVID-19 virus wild type may include an amino acid sequence represented by SEQ ID NO: 136, the RBD of the COVID-19 virus alpha variant may include an amino acid sequence represented by SEQ ID NO: 139, the RBD of the COVID-19 virus beta variant may include an amino acid sequence represented by SEQ ID NO: 140, the RBD of the COVID-19 virus gamma variant may include an amino acid sequence represented by SEQ ID NO: 141, the RBD of the COVID-19 virus delta variant may include an amino acid sequence represented by SEQ ID NO: 142, the COVID-19 virus kappa variant may include an amino acid sequence represented by SEQ ID NO: 143, and the RBD of the COVID-19 virus mu variant may include an amino acid sequence represented by SEQ ID NO: 144.

The antibody includes not only a whole antibody, but also a functional fragment of the antibody molecule. The whole antibody has a structure with two full-length light chains and two full-length heavy chains, and each light chain is linked to heavy chain by disulfide bond. The functional fragment of an antibody molecule indicates a fragment retaining a antigen-binding function, and examples of the antibody fragment include (i) Fab fragment consisting of light chain variable region (VL) and heavy chain variable region (VH), and light chain constant region (CL) and heavy chain 1st constant region (CH1); (ii) Fd fragment consisting of VH and CH1 domains; (iii) Fv fragment consisting of VL and VH domains of a monoclonal antibody; (iv) dAb fragment consisting of VH domain (Ward ES et al., Nature 341:544-546 (1989)); (v) separated CDR region; (vi) F(ab')2 fragment including two linked Fab fragments, as a divalent fragment; (vii) single chain Fv molecule (scFv) in which VH and VL domains are linked by a peptide linker to form an antigen binding site; (viii) bi-specific single chain Fv dimmer (PCT/US92/09965), (ix) multivalent or multi-specific diabody fragment (WO94/13804) prepared by gene fusion, and the like.

As used herein, the antibody or immunologically active fragment thereof may be selected from the group consisting of animal-derived antibody, chimeric antibody, humanized antibody, human antibody, and immunologically active fragment thereof. The antibody may be recombinantly or synthetically produced.

When an animal-derived antibody produced by immunizing a desired antigen to an unimmunized animal is administered to a human for a therapeutic purpose, immunorejection may generally occur. In order to inhibit the immunorejection, a chimeric antibody has been developed. In the chimeric antibody, the constant region of animal-derived antibody causing an anti-isotype reaction is replaced with the constant region of human antibody by genetic engineering. The chimeric antibody has been significantly improved in terms of the anti-isotype reaction compared to the animal-derived antibody, but still, it has potential side effects of anti-idiotypic reaction, since animal-derived amino acids are present in a variable region. The humanized antibody is developed for improving these side effects. This is constructed by grafting a CDR (complementarity determining region) playing an important role for binding of antigen in variable region of chimeric antibody into a human antibody framework.

The most important thing for the CDR grafting technology for constructing the humanized antibody is selecting an optimized human antibody which may receive the CDR of animal-derived antibody at best, and for this, the utilization of antibody database, analysis of crystal structure, molecular modeling technology, etc. are utilized. However, the application of additional antibody engineering technology for restoring antigen binding activity may be essential, since amino acids located on the framework of animal-derived antibody may affect the antigen binding, despite of grafting the CDR of animal-derived antibody into optimized human antibody framework, and therefore there are many cases in which the antigen binding activity cannot be conserved.

The antibody or immunologically active fragment thereof may be isolated from a living body (not present in a living body) or may be non-naturally occurring, for example, may be synthetically or recombinantly produced.

As used herein, the term "antibody" means a material produced by stimuli of antigen in an immune system, and its kind is not particularly limited, and may be obtained naturally or non-naturally (for example, synthetically or recombinantly). The antibody is advantageous for massive expression and production, since it is very stable *in vitro* and *in vivo,* and its half-life is long. In addition, the antibody has a dimer structure, and therefore, its avidity is very high. The complete antibody has a structure composed of two full length light chains and two full length heavy chains, and each light chain is linked to the heavy chain by disulfide bonds. The constant region of antibody is divided into the heavy chain constant region and light chain constant region, and the heavy chain constant region has gamma (γ), mu (µ), alpha (α), delta (δ) and epsilon (ε) types and has gamma1 (γ1), gamma2 (γ2), gamma3 (γ3), gamma4 (γ4), alpha1 (α1) and alpha2 (α2) as subclasses. The constant region of light chain has kappa (κ) and lambda (λ) types.

As used herein, the term "heavy chain" is interpreted as including all of full length heavy chains including variable region domain V_{H} including the amino acid sequence containing variable region sequence sufficient for giving specificity to an antigen, 3 constant region domains C_{H1}, C_{H2} and C_{H3} and hinge, and fragments thereof. In addition, the term, "light chain" is interpreted as including all of full length light chains including variable region domain V_{L} including the amino acid sequence containing variable region sequence sufficient for giving specificity to an antigen, and constant region domain C_{L}, and fragments thereof.

As used herein, the term "variable region" or "variable domain" refers to a portion of an antibody molecule that exhibits many variations on the sequence while performing a function that specifically binds to an antigen, and in the variable region, there are the complementarity determining regions CDR1, CDR2, and CDR3. A framework region (FR) portion exists between the CDRs to support a CDR ring. The "complementarity determining region" is a ring-shaped region involved in antigen recognition, and the specificity of the antibody for the antigen is determined as the sequence of this region changes.

As used herein, the term "single chain fragment variable (scFv)" refers to a single chain antibody formed by expressing only a variable region of the antibody through genetic recombination, and means an antibody in a single chain form in which the VH and VL regions of the antibody are linked to each other by a short peptide chain. The term "scFv" is intended to include an scFv fragment including an antigen-binding fragment, unless otherwise specified or otherwise understood from the context. This is obvious to those skilled in the art.

As used herein, the term "complementarity determining region (CDR)" refers to an amino acid sequence of a hypervariable region of the heavy and light chains of immunoglobulin. The heavy and light chains may include three CDRs (CDRH1, CDRH2, CDRH3 and CDRL1, CDRL2, CDRL3), respectively. The CDRs may provide key contact residues for the antibody binding to an antigen or epitope.

As used herein, the term "specifically binding" or "specifically recognizing" has the same meaning as commonly known to those skilled in the art, and means that an antigen and an antibody specifically interact with each other to cause an immunological response.

As used herein, the term "antigen-binding fragment" refers to a fragment of the entire structure of immunoglobulin, and refers to a portion of a polypeptide including a portion capable of binding with the antigen. For example, the antigen-binding fragment may be scFv, (scFv)2, scFv-Fc, Fab, Fab' or F(ab')2, but is not limited thereto. The Fab of the antigen-binding fragments has a structure having variable regions of the light and heavy chains, a constant region of the light chain, and a first constant region (C_{H1}) of the heavy chain, and has one antigen binding domain. The Fab' is different from Fab in that there is a hinge-region containing one or more cysteine residues at a C-terminal of the heavy chain C_{H1} domain. The F(ab')2 antibody is produced when the cysteine residues in the hinge region of Fab' form disulfide bonds. Fv is a minimal antibody fragment having only a heavy chain variable region and a light chain variable region, and a recombination technique for generating the Fv fragment is widely known in the art. The two-chain Fv has the heavy chain variable region and the light chain variable region linked via a noncovalent bond, and the single-chain Fv may generally form a dimer-like structure, such as the two-chain Fv because the variable region of the heavy chain and the variable region of the single chain are covalently linked via a peptide linker or directly linked at a C-terminal. The linker may be a peptide linker consisting of 1 to 100 or 2 to 50 random amino acids, and appropriate sequences are known in the art. The antigen-binding fragments may be obtained using protease (for example, the whole antibody is restriction-cleaved with papain to obtain Fab, and cleaved with pepsin to obtain an F(ab')2 fragment), and may be produced through genetic recombination technology.

As used herein, the term "hinge region" refers to a region included in the heavy chain of the antibody, and means a region which is present between the C_{H1} and C_{H2} regions and functions to provide flexibility of the antigen binding domain in the antibody. For example, the hinge may be derived from a human antibody, specifically, derived from IgA, IgE, or IgG, such as IgG1, IgG2, IgG3, or IgG4.

In one aspect, an embodiment of the present invention relates to an isolated nucleic acid molecule encoding the antibody or the immunologically active fragment thereof of an embodiment of the present invention, a vector including the isolated nucleic acid molecule, and a host cell transformed with the vector.

The nucleic acid molecule of an embodiment of the present invention may be isolated or recombinant, and includes not only DNA and RNA in single-chain and two-chain form, but also corresponding complementary sequences. The isolated nucleic acid is a nucleic acid that has been isolated from surrounding genetic sequences present in the genome of a subject from which the nucleic acid was isolated, in the case of a nucleic acid isolated from a naturally occurring source. In the case of a nucleic acid synthesized enzymatically or chemically from a template, such as a PCR product, a cDNA molecule, or an oligonucleotide, the nucleic acid produced from such a procedure may be understood as an isolated nucleic acid molecule. The isolated nucleic acid molecule refers to a nucleic acid molecule in the form of a separate fragment or as a component of a larger nucleic acid construct. The nucleic acid is operably linked when placed in a functional relationship with another nucleic acid sequence. For example, DNA of a pre-sequence or secretory leader is expressed as a preprotein in the form before the polypeptide is secreted to be operably linked to DNA of the polypeptide, and a promoter or enhancer is operably linked to a coding sequence when affecting the transcription of the polypeptide sequence, or a ribosome binding domain is operably linked to a coding sequence when disposed to promote the translation. In general, the operably linked means that DNA sequences to be linked are located contiguously, and that the secretory leader exists contiguously in the same reading frame. However, the enhancer needs not to be contiguously located. The linkage is achieved by ligation at a convenient restriction enzyme site. When there is no such site, synthetic oligonucleotide adapters or linkers are used according to conventional methods.

In the isolated nucleic acid molecule encoding the antibody or the immunologically active fragment thereof of the present invention due to the degeneracy of codons or in consideration of codons preferred in an organism in which the antibody is to be expressed, it will be well understood to those skilled in the art that various modifications may be made in a coding region within a range without changing the amino acid sequence of the antibody to be expressed from the coding region, various modifications or changes may be made within a range without affecting the expression of the gene even in parts other than the coding region, and such modified genes are also included within the scope of the present invention. That is, as long as the nucleic acid molecule of the present invention encodes a protein having equivalent activity thereto, one or more nucleobases may be mutated by substitution, deletion, insertion, or a combination thereof, which are also included in the scope of the present invention. The sequence of such a nucleic acid molecule may be single- or two-chain, and may be a DNA molecule or an RNA (mRNA) molecule.

According to an embodiment of the present invention, the isolated nucleic acid molecule encoding the antibody or the immunologically active fragment thereof of the present invention may be inserted into an expression vector for protein expression. The expression vector generally includes a protein operably linked, i.e., functionally related with a control or regulatory sequence, a selectable marker, an optional fusion partner, and/or an additional element. In appropriate conditions, the antibody or the immunologically active fragment thereof of the present invention may be produced by a method of inducing the protein expression by culturing a host cell transformed with a nucleic acid, preferably an expression vector containing an isolated nucleic acid molecule encoding the antibody or the immunologically active fragment thereof of the present invention. A variety of suitable host cells including mammalian cells, bacteria, insect cells, and yeast may be used, but are not limited thereto. Methods for introducing exogenous nucleic acids into host cells are known in the art and will vary depending on a host cell to be used. Preferably, it is possible to produce *E. coli,* which has high industrial value due to low production cost, as a host cell.

The vector of an embodiment of the present invention may include a plasmid vector, a cosmid vector, a bacteriophage vector, a viral vector, etc., but is not limited thereto. The suitable vector includes a signal sequence or a leader sequence for membrane targeting or secretion in addition to expression regulatory elements such as a promoter, an operator, an initiation codon, a termination codon, a polyadenylation signal, and an enhancer and may be variously produced according to a purpose. The promoter of the vector may be constitutive or inductive. The signal sequence may use a PhoA signal sequence, an OmpA signal sequence, etc. in the case of *Escherichia* sp. bacteria as a host, an α-amylase signal sequence, a subtilisin signal sequence, etc. in the case of *Bacillus* sp. bacteria as a host, an MFα signal sequence, an SUC2 signal sequence, etc. in the case of yeast as a host, and an insulin signal sequence, an α-interferon signal sequence, an antibody molecule signal sequence, etc. in the case of an animal cell as a host, but is not limited thereto. Further, the vector may include a selectable marker for selecting a host cell including a vector and a replicable expression vector includes a replication origin.

As used herein, the term "vector" refers to a vehicle into which a nucleic acid sequence may be inserted for introduction into a cell capable of replicating the nucleic acid sequence. The nucleic acid sequence may be exogenous or heterologous. The vector may include plasmids, cosmids, and viruses (for example, bacteriophage), but is not limited thereto. Those skilled in the art may construct vectors by standard recombinant techniques (Maniatis, et al., Molecular Cloning, A Laboratory Manual, Cold Spring Harbor Press, Cold Spring Harbor, N.Y., 1988; and Ausubel et al., In: Current Protocols in Molecular Biology, John, Wiley & Sons, Inc, NY, 1994, etc.).

In an embodiment, when constructing the vector, expression regulatory sequences such as a promoter, a terminator, and an enhancer, sequences for membrane targeting or secretion, etc. may be appropriately selected according to a type of host cell intended to produce the antibody and may be variously combined depending on a purpose.

As used herein, the term "expression vector" refers to a vector including a nucleic acid sequence encoding at least a portion of a gene product to be transcribed. In some cases, the RNA molecule is then translated into a protein, a polypeptide, or a peptide. The expression vector may include various regulatory sequences. In addition to regulatory sequences that regulate transcription and translation, vectors and expression vectors may also include nucleic acid sequences that serve other functions.

As used herein, the term "host cell" includes a eukaryote and a prokaryote, and refers to any transformable organism capable of replicating the vector or expressing a gene encoded by the vector. The host cell may be transfected or transformed by the vector, which means a process in which an exogenous nucleic acid molecule is delivered or introduced into the host cell.

In an embodiment, the host cell may be a bacterial or animal cell, the animal cell line may be a CHO cell, an HEK cell or an NSO cell, and the bacteria may be *E. coli.*

In one aspect, an embodiment of the present invention relates to a method of preparing an antibody specific to COVID-19 virus or a variant thereof or an immunologically active fragment thereof, in which the method includes: a) culturing a host cell including a vector including the isolated nucleic acid molecule; and b) recovering the antibody or the immunologically active fragment thereof from host cell culture.

In an embodiment, after the recovering of the antibody or the immunologically active fragment thereof from the host cell culture, purifying the antibody or the immunologically active fragment thereof may be further included.

The antibody or the immunologically active fragment thereof of an embodiment of the present invention may be isolated or purified by various methods known in the art. Standard purification methods include chromatographic techniques, electrophoresis, immunoprecipitation, dialysis, filtration, concentration, and chromatofocusing techniques. As is well known in the art, a variety of natural proteins such as bacterial proteins A, G, and L may bind to antibodies, and thus these proteins may be used for the purification of antibodies. Purification may often be enabled by using a particular fusion partner.

The vector according to an embodiment of the present invention may be transformed into a suitable host cell, for example, *E. coli* or yeast cell, and the transformed host cell may be incubated to produce an antibody or an immunologically active fragment thereof according to an embodiment of the present invention in mass quantities. Incubation methods and media conditions suitable for a kind of host cell may be easily chosen from those known to those skilled in the art. The host cell may be a prokaryote such as *E. coli* or *Bacillus subtilis.* In addition, it may be a eukaryotic cell derived from a yeast such as *Saccharomyces cerevisiae,* an insect cell, a plant cell, and an animal cell. More preferably, the animal cell may be an autologous or allogeneic animal cell. A transformant prepared through introduction into an autologous or allogeneic animal cell may be administered to a subject for use in cellular therapy against cancer. As for a method for introducing an expression vector into the host cell, it is possible to use any method if it is known to those skilled in the art. Other organisms, including transgenic (for example, genetically engineered) mice, or other mammals, may be used to produce the antibody or the immunologically active fragment thereof of an embodiment of the present invention (for example, *see* US 6,300,129). For example, it is known that only the variable regions of mouse immune genes (heavy chain V, D, and J segments, and light chain V and J segments) are replaced with the corresponding human variable sequences, so that engineered mice may be used to mass produce high-affinity antibodies with human variable sequences (for example, *see* US 6,586,251; US 6,596,541, and US 7,105,348).

In one aspect, an embodiment of the present invention relates to a pharmaceutical composition for preventing or treating COVID-19 virus infection containing an antibody specific to COVID-19 virus or a variant thereof or an immunologically active fragment thereof of an embodiment of the present invention.

In an embodiment, the COVID-19 virus infection may also be referred to as novel coronavirus infection or COVID-19.

In an embodiment, the COVID-19 virus infection may be caused by infection with a COVID-19 virus (SARS-CoV-2) wild type, a COVID-19 virus alpha variant, a COVID-19 virus beta variant, a COVID-19 virus gamma variant, a COVID-19 virus delta variant, a COVID-19 virus kappa variant, or a COVID-19 virus mu variant.

As used herein, the term "prevention" refers to all actions that inhibit or delay the occurrence, spread, and recurrence of COVID-19 virus infection by administering the composition according to an embodiment of the present invention.

As used herein, the term "treatment" refers to all actions that alleviate or beneficially change the symptoms of COVID-19 virus infection and complications resulting therefrom by administering the composition of an embodiment of the present invention. Those skilled in the art to which the present invention pertains will identify the exact criteria of a disease in which the composition of an embodiment of the present invention is effective and determine the degree of alleviation, improvement and treatment with reference to the data presented by the Korean Medical Association, etc.

As used herein, the term "therapeutically effective amount" used in combination with the active ingredient in an embodiment of the present invention refers to an amount effective to prevent or treat COVID-19 virus infection, and the therapeutically effective amount of the composition of an embodiment of the present invention may be determined by various factors, for example, administration method, target site, the patient's condition, and the like. Accordingly, the dosage when used in the human body should be determined in appropriate amounts in consideration of safety and efficacy. It is also possible to estimate the amount used in humans from the effective amount determined by animal experiments. These matters to be considered in determining the effective amount are described in, for example, Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The composition of an embodiment of the present invention is administered in a pharmaceutically effective amount. The term "pharmaceutically effective amount" used herein refers to an amount sufficient to treat the disease at a reasonable benefit/risk ratio applicable for medical treatment and an amount that does not cause side effects. The level of an effective dosage may be determined by parameters including a health status of the patient, severity of COVID-19 virus infection, the activity of a drug, sensitivity to a drug, an administration method, administration time, an administration route and a release rate, duration of treatment, formulated or co-used drugs, and other parameters well known in medical fields. The composition of an embodiment of the present invention may be administered as an individual therapeutic agent or in combination with other therapeutic agents. It may be administered sequentially or simultaneously with a conventional therapeutic agent or administered in a single- or multiple-dose regime. In consideration of all of the above parameters, it is important to administer such a dose to obtain a maximum effect with a minimal amount without a side effect and the dose may be easily determined by those skilled in the art.

The pharmaceutical composition of an embodiment of the present invention may include carriers, diluents, excipients, or a combination of two or more thereof commonly used in biological formulations. The term "pharmaceutically acceptable" as used herein means that the composition is free of toxicity to cells or humans exposed to the composition. The carrier is not particularly limited as long as it is suitable for the delivery of the composition to the living body. For example, compounds, saline solutions, sterile water, Ringer's solution, buffered saline, dextrose solution, maltodextrin solution, glycerol, ethanol disclosed in Merck Index, 13th ed., Merck & Co. Inc. and one or more ingredients thereof may be mixed and used. If necessary, conventional additives such as antioxidants, buffers, and bacteriostatic agents may be added. The composition may also be prepared into dosage form for injection such as aqueous solution, suspension, or emulsion, tablet, capsule, powder or pill by additionally including diluents, dispersant, surfactant, binder and lubricant. Further, the composition may be formulated into a desirable form depending on targeting disease or ingredients thereof, using the method disclosed in Remington's Pharmaceutical Science (Mack Publishing Company, Easton Pa., 18th, 1990).

In an embodiment, the pharmaceutical composition may be one or more formulations selected from the group consisting of oral formulations, external preparations, suppositories, sterile injectable solutions and sprays, and more preferably oral formations or injectable formulations.

As used herein, the term "administration" means providing a predetermined substance to a subject or a patient by any appropriate method and may be administered orally or parenterally (for example, by applying in injectable formulations intravenously, subcutaneously, intraperitoneally, or topically). The dosage may vary depending on the patient's body weight, age, gender, health condition, diet, administration time, administration method, excretion rate, the severity of the disease, and the like. The liquid formulations for oral administration of the composition of an embodiment of the present invention include suspensions, oral liquids, emulsions, syrups and the like. In addition to water and liquid paraffin which are simple diluents commonly used, various excipients such as wetting agents, sweeteners, flavors, preservatives and the like may be included. Formulations for parenteral administration include sterile aqueous solutions, non-aqueous solvents, suspensions, emulsions, freeze-dried formulations, suppositories, and the like. The pharmaceutical composition of an embodiment of the present invention may be administered by any device capable of moving the active substance to target cells. The preferred administration method and formulations include intravenous, subcutaneous, intradermal, intramuscular, drip injections and the like. The injectable solution may be prepared using an aqueous solvent such as a physiological saline solution and Ringer's solution and a non-aqueous solvent such as a vegetable oil, a higher fatty acid ester (for example, ethyl oleate), an alcohol (for example, ethanol, benzyl alcohol, propylene glycol, glycerin, etc.) and may include pharmaceutical carriers such as stabilizer to prevent deterioration (for example, ascorbic acid, sodium hydrogen sulfite, sodium pyrosulfite, BHA, tocopherol, EDTA, etc.), an emulsifier, a buffer for pH control, preservatives for inhibition of microbial growth (for example, phenylmercuric nitrate, thimerosal, benzalkonium chloride, phenol, cresol, benzyl alcohol, etc.).

As used herein, the term "subject" means all animals who have developed the COVID-19 virus infection or are capable of developing the COVID-19 virus infection, including human, a monkey, a cow, a horse, sheep, a pig, a chicken, a turkey, a quail, a cat, a dog, a mouse, a bat, a camel, a rat, a rabbit or a guinea pig, and the term "specimen" may be droplets, sputum, whole blood, plasma, serum, urine or saliva isolated therefrom.

The pharmaceutical composition of an embodiment of the present invention may further include a pharmaceutically acceptable additive, which is exemplified by starch, gelatinized starch, microcrystalline cellulose, milk sugar, povidone, colloidal silicon dioxide, calcium hydrogen phosphate, lactose, mannitol, taffy, Arabia rubber, pregelatinized starch, corn starch, cellulose powder, hydroxypropyl cellulose, Opadry, sodium starch glycolate, carnauba wax, synthetic aluminum silicate, stearic acid, magnesium stearate, aluminum stearate, calcium stearate, white sugar, dextrose, sorbitol, talc, etc. The pharmaceutically acceptable additive of an embodiment of the present invention is preferably added to the composition in an amount of 0.1 to 90 parts by weight but is not limited thereto.

In one aspect, an embodiment of the present invention relates to a composition for detecting COVID-19 virus or a variant thereof, in which the composition includes an antibody specific to the COVID-19 virus or the variant thereof or an immunologically active fragment thereof.

The antibodies or the immunologically active fragments thereof used in the detection (diagnostic) composition of an embodiment of the present invention are preferably labeled so as to be detectable. A variety of methods available for labeling biomolecules are well known to those skilled in the art and are considered within the scope of an embodiment of the present invention. The methods are described in Tijssen, 'Practice and theory of enzyme immuno assays', Burden, RHand von Knippenburg (Eds), Volume 15 (1985), 'Basic methods in molecular biology'; Davis LG, Dibmer MD; Battey Elsevier (1990), Mayer et al., (Eds) 'Immunochemical methods in cell and molecular biology' Academic Press, London (1987), or in the series 'Methods in Enzymology', Academic Press, Inc.

There are labeling methods and many other labels known to those skilled in the art. Examples of types of labels that may be used in an embodiment of the present invention include enzymes, radioisotopes, colloidal metals, fluorescent compounds, chemiluminescent compounds, and bioluminescent compounds.

Labels that are commonly used include fluorescent substances (for example, fluorescein, rhodamine, Texas Red, etc.), enzymes (for example, horse radish peroxidase, β-galactosidase, alkaline phosphatase), radioactive isotopes (for example, ³²P or ¹²⁵I), biotin, digoxygenin, colloidal metals, chemi- or bioluminescent compounds (for example, dioxetanes, luminol or acridiniums). Labeling procedures, such as covalent coupling of enzymes or biotinyl groups, iodinations, phosphorylations, biotinylations, etc., are well known in the art.

Detection methods include, but are not limited to, autoradiography, fluorescence microscopy, direct and indirect enzymatic reactions, etc. Detection assays that are commonly used include radioisotopic or non-radioisotopic methods. These assays include, inter alia, RIA (radioimmuno assay) and IRMA (immune radioimmunometric assay), EIA (enzyme immuno assay), ELISA (enzyme linked immuno sorbent assay), FIA (fluorescent immuno assay) and CLIA (chemioluminescent immune assay).

In one aspect, an embodiment of the present invention relates to a kit for detecting COVID-19 virus or a variant thereof, in which the kit includes a composition for detecting the COVID-19 virus or the variant thereof.

In an embodiment, the kit may include a composition for detecting the COVID-19 virus or the variant thereof according to an embodiment of the present invention and a reagent for detecting an antigen-antibody complex. The reagent for detecting the antigen-antibody complex includes reagents for radioimmunoassay, ELISA (enzyme linked immunesorbent assay), or immunofluorescence analysis.

In an embodiment, the detection of the antigen-antibody complex may be achieved using an Ouchterlony plate simply detecting the antibody and/or antigen via the antigen-antibody binding, western blot, and immuno electrophoresis such as Crossed IE, Rocket IE, Fused Rocket IE, and Affinity IE. Reagents or substances as used in this method are known. This may be detected, for example, via antigen-antibody reactions, or substrates, nucleic acids or peptide aptamers that specifically bind to antigens, or reactions with receptors, ligands, or cofactors interacting with the complex or using mass spectrometry. The reagent or substance that specifically interacts or binds to the antigen-antibody complex of the present application may be used in a chip method or in combination with nanoparticles. The immunoassay or immunostaining method is described in Enzyme Immunoassay, E. T. Maggio, ed., CRC Press, Boca Raton, Fla., 1980; Gaastra, W., Enzyme-linked immunosorbent assay (ELISA), in Methods in Molecular Biology, Vol. 1, Walker, J. M. ed., Humana Press, N J, 1984, etc. Analyzing the intensity of the final signal by the above-described immunoassay process, that is, performing signal contrast with a normal sample may diagnose absence or presence of the infection of the COVID-19 virus by detecting the COVID-19 virus in a subject.

In one aspect, an embodiment of the present invention relates to a method for detecting COVID-19 virus or a variant thereof, in which the method includes: (a) contacting a sample separated from a specimen with an antibody specific to the COVID-19 virus or the variant thereof or the immunologically active fragment thereof of an embodiment of the present invention; and (b) detecting a formation of an antigen-antibody complex.

In an embodiment, the variant of the COVID-19 virus may be an alpha variant, a beta variant, a gamma variant, a delta variant, a kappa variant, or a mu variant.

In one aspect, an embodiment of the present invention relates to a method of providing information regarding diagnosis of COVID-19 virus infection, in which the method includes: (a) contacting a sample separated from a specimen with an antibody specific to the COVID-19 virus or the variant thereof or the immunologically active fragment thereof of an embodiment of the present invention to form an antigen-antibody complex; and (b) detecting a formation of the complex.

In an embodiment, the COVID-19 virus infection may be caused by infection with a COVID-19 virus (SARS-CoV-2) wild type, a COVID-19 virus alpha variant, a COVID-19 virus beta variant, a COVID-19 virus gamma variant, a COVID-19 virus delta variant, a COVID-19 virus kappa variant, or a COVID-19 virus mu variant.

Hereinafter, the present invention will be described in more detail with reference to the following Examples. However, the following Examples are only intended to embody the contents of the present invention, and the present invention is not limited thereto.

### [Modes for Carrying Out the Invention]

### Example 1. Production of COVID-19 Virus RBD (Receptor Binding Domain) Antigen

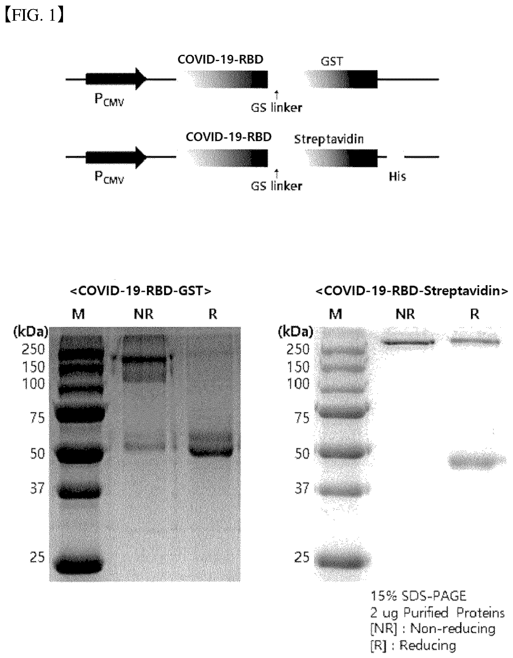

In order to create an antibody that binds to an RBD (receptor binding domain) region, which is known to be important for the virus to infect the host by binding to the surface of the ACE2 (angiotensin converting enzyme 2) receptor present on the host cells, the RBD region (SEQ ID NO: 136) of COVID-19 virus was prepared as an antigen. In addition, in order to produce an antigen more suitable for screening an antibody using a flow cytometer, a vector for animal cell expression was constructed to produce antigens in a dimeric form fused with a GST tag and a tetramer form fused with a streptavidin tag (FIG. 1). After mixing 30 ml of Freestyle 293 expression culture solution (Gibco, 12338-018) with each vector, a solution of PEI (polyethylenimine) (Polyscience, 23966) and each vector mixed at a 4 : 1 ratio was left at room temperature for 20 minutes and then treated and transfected into 300 ml of Expi293F animal cells subcultured the previous day at a density of 2 × 10⁶ cells/ml. Thereafter, the transfected cells were incubated for 7 days under conditions of 37°C, 125 rpm, and 8 % CO₂ in a shaking CO₂ incubator, and then centrifuged to collect only a supernatant. Thereafter, the supernatant was equilibrated with 25x PBS and filtered through a 0.2 µm filter (Merck Millipore) using a bottle top filter. The filtered culture solution was added with 1 ml of GSH resin and Ni-NTA resin and stirred at 4°C for 16 hours, and then the resin was recovered through the column, washed with 5 ml PBS and eluted with 4 ml of 50 mM Tris-HCl & 10 mM reduced glutathione (SIGMA) at pH 8.0 and 250 mM imidazole. The buffer was replaced with PBS using centrifugal filter units 3K (Merck Millipore), and then the size and purity of two purified antigen proteins (COVID-19-RBD-GST represented by SEQ ID NO: 137 and COVID-19-RBD-Streptavidin represented by SEQ ID NO: 138) were analyzed by SDS-PAGE under non-reducing conditions (NR) and reducing conditions (R), respectively (FIG. 1).

### Example 2. Human Antibody Screening using Dimeric Form of COVID-19 Virus-RBD

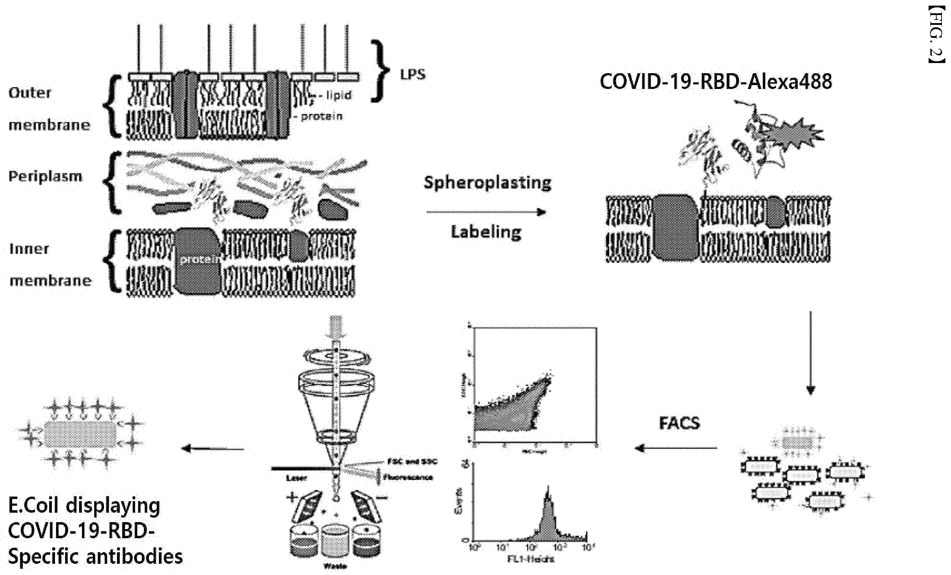

Among the antigen proteins produced in the above example, the GST-fused antigen protein was labeled with the Alexa488 fluorescent molecule for use in screening using a flow cytometer. Thereafter, screening for human antibodies binding to the COVID-19 virus-RBD region was performed using the bacterial displayed human scFv antibody library constructed by the present inventors. Specifically, 1 ml of library cells were cultured in 25 ml of TB medium containing 2% glucose and 40 µg/ml of chloramphenicol at 37°C and 250 rpm for 4 hours, and the cultured cells were cultured in 100 ml of TB medium containing 40 µg/ml of chloramphenicol at a ratio of 1 : 100 to OD600 = 0.5. After cooling at 25°C and 250 rpm for 20 minutes, then adding 1 mM IPTG and induction for 5 hours at 25°C and 250 rpm, and *E. coli* overexpressing the protein was harvested based on OD600 = 8. Thereafter, in order to make the cells into spheroplasts suitable for screening using a flow cytometer, the cells were resuspended using 1 ml of 10 Mm Tris-HCl (pH 8.0), washed twice, and the remaining medium was removed. The outer cell membrane was removed by applying osmotic shock by rotating the cells in 1 ml of STE [0.5 M sucrose, 10 Mm Tris-HCl, 10 Mm EDTA (pH 8.0)] solution at 37°C for 30 minutes. Thereafter, the peptidoglycan layer was removed by rotating a mixed solution of 1 ml of Solution A and 20 µl of 50 mg/ml lysozyme solution for 15 minutes at 37°C, and washed with 1 ml of PBS. 700 µl of PBS and 200 nM (monomer basis) of the COVID-19-RBD-GST-Alexa488 probe were added to 300 µl of the mixed solution and rotated at room temperature for 1 hour to label the spheroplasts with the fluorescent probe. Thereafter, clones showing high fluorescence due to increased antigen binding activity were recovered using a flow cytometer (FIG. 2).

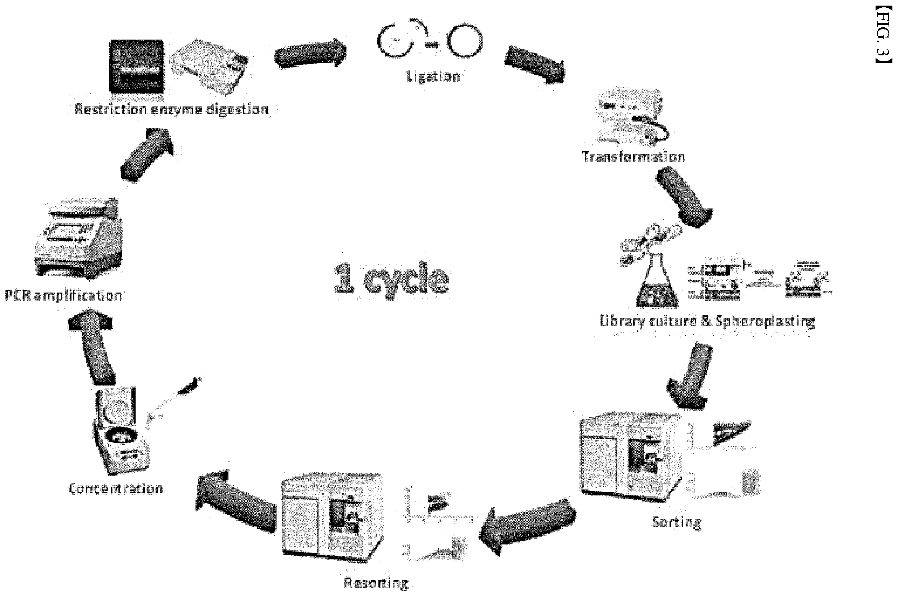

The process of selecting spheroplasts showing high affinity to the COVID-19 virus-RBD region was repeated through amplification of scFv genes from recovered clones using a PCR method, culture, expression induction, a spheroplasting process to remove an outer cell membrane and peptidoglycan layer, antigen labeling, and selective gating of flow cytometry (FIG. 3). After the selection process using a flow cytometer and the re-selection process to increase selection purity, the next round of selection process was performed to amplify (enrich) the desired clones after going through an enrichment process, a process of securing selected scFv variant genes through PCR amplification, a subcloning process, and a transformation process. Through this repeated search process of several rounds, scFv variant clones with excellent binding activity to the COVID-19 virus-RBD region were secured.

### Example 3. Identification of Genetic Sequence of COVID-19 Binding Antibody Variant

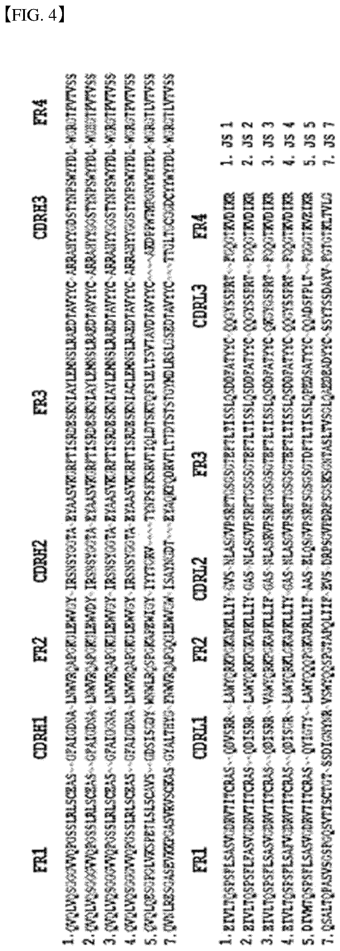

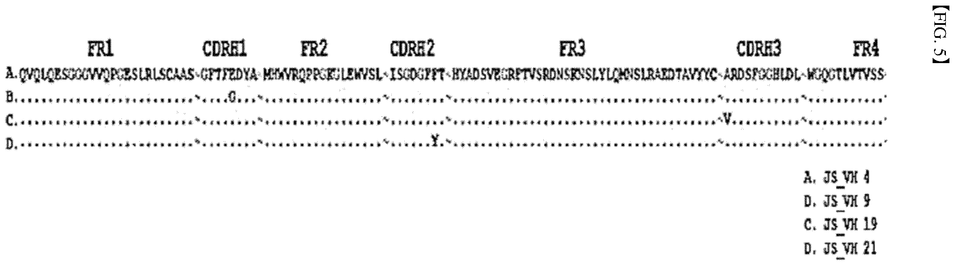

In order to identify the gene sequences of the scFv variant clones obtained in Example 2 above, the DNA base sequences were analyzed using Sanger sequencing. Through analysis, six types of scFv antibody variants with antibody sequences were selected. In this process, four types of VH antibody variants with increased binding activity to the COVID-19 virus-RBD region were additionally selected only through the VH of scFv. In addition, the VH amino acid sequences and VL amino acid sequences of six types of scFv variants (FIG. 4) and the VH amino acid sequences of four types of VH variants (FIG. 5) were identified (Table 1).

**[Table 1]**

| **Variants** | **Regions** | **Sequences** | **SEQ ID NO** |
|---|---|---|---|
| JS 1 | VH | QVQLVQSGGG VVQPGSSLRL SCEASGFAIG DNALNWVRQA PGKGLEWVGY IRSNSYGGTAEYAASVKGRF TISRDESKNI AYLEMNSLRA EDTAVYYCAR RAHYYGDSTY NPSWYFDLWGRGTPVTVSS | 105 |
| | VL | E VLTQSPSF LSASVGDRVT ITCRASQDVS RRLAWYQRKP GKAPKLLIYG VSNLASGVPSRFTGSGSGTE FTLTISSLQS DDFATYYCQQ GYSSPRTFGQ GTKVDIKR | 121 |
| JS 2 | VH | QVQLVQSGGG VVQPGSSLRL SCEASGFAIG DNALNWVRQA PGKGLEWVDY IRSNSYGGTAEYAASVKGRF TISRDESKNI AYLEMNSLRA EDTAVYYCAR RAHYYGGSTY NPSWYFDLWGHGTPVTVSS | 106 |
| | VL | E[?]VLTQSPSF LFASVGDRVT ITCRASQDIS RRLAWYQRKP GKAPKLLIYG ASNLASGVPSRFTGSGSGTE FTLTISSLQS DDFATYYCQQ GYSSPRTFGQ GTKVDIKR | 122 |
| JS 3 | VH | QVQLVQSGGG VVQPGSSLRL SCEASGFAIG GNALNWVRQA PGKGLEWVGY IRSNSYGGTAEYAASVKGRF TISRDESKNI AYLEMNSLRA EDTAVYYCAR RAHYYGGSTY NPSWYFDLWGRGTPVTVSS | 107 |
| | VL | EIVLTQSPSF LSASVGDRVT ITCRASQDIS RRLAWYQRKP GKAPKLLIFG ASNLASRVPSRFTGSGSGTE FTLTISSLQS DDFATYYCQQ GYGSPRTFGQ GTKVDIKR | 123 |
| JS 4 | VH | QVQLVQSGGG VVQPGSSLRL SCEASGFAIG DNALNWVRQA PGKGLEWVGY IRSNSYGGTAEYAASVKGRF TISRDESKNI ACLEMNSLRA EDTAVYYCAR RAHYYGGSTY NPSWYFDLWGRGTPVTVSS | 108 |
| | VL | [?]VLTQSPSF LSAFVGDRVT ITCRASQDIS GRLAWYQRKL GKAPKLLIYG ASNLASGVPSRFTGSGSGTE FTLTISSLQS DDFATYYCQQ GYSSPRTFGQ GTKVDIKR | 124 |
| JS 5 | VH | QVQLQESGPG LVKSPETLSL SCAVSGDSIS GDYWNWLRQS PGKAPEWIGY IYYTGRVTYN PSFKSRVTIQ LDTSKTQFSL ELTSVTAVDT AVYYCAKDPP WTMPGNYWYF DLWGRGTLVTVSS | 109 |
| | VL | DIVMTQSPSF LSASVGDRVT TCRASQYIG TYLAWYQQQP GKAPRLLIFA ASELQSGVPSRFSGSGSGTD FTLTISSLQP EDSATYYCQQ ADSFPLTFGG GTKVEIKR | 123 |
| JS 7 | VH | QVNLRESGAS EVKKPGASVR VSCKASGYAL THYGFNWVRQ APGQGLEWVG WISAYNGDTE YAQKFQDRVT LTTDTSTSTG YMDLRSLGSE DTAVYYCTTG LTGCGGDCYY WYFDLWGRGT LVTVSS | 110 |
| | VL | QSALTQPASV SGSPGQSVTI SCTGTSSDIG NYNRVSWYQQ SPGTAPQLII FEVSDRPSGV PDRFSGSKSG NTASLTVSGL QAEDEADYYC SSYTSSDAYV FGTGTKLTVL G | 126 |
| JS_VH 4 | VH | QVQLQESGGG VVQPGESLRL SCAASGFTFE DYAMHWVRQP PGKGLEWVSL ISGDGFFTHY ADSVEGRFTV SRDNSKNSLY LQMNSLRAED TAVYYCARDS FGGHLDLWGQ GTLVTVSS | 111 |
| JS_VH 9 | VH | QVQLQESGGG VVQPGESLRL SCAASGFTFG DYAMHWVRQP PGKGLEWVSL ISGDGFFTHYADSVEGRFTV SRDNSKNSLY LQMNSLRAED TAVYYCARDS FGGHLDLWGQ GTLVTVSS | 112 |
| JS_VH 19 | VH | QVQLQESGGG VVQPGESLRL SCAASGFTFE DYAMHWVRQP PGKGLEWVSL ISGDGFFTHY ADSVEGRFTV SRDNSKNSLY LQMNSLRAED TAVYYCVRDS FGGHLDLWGQ GTLVTVSS | 113 |
| JS_VH 21 | VH | QVQLQESGGG VVQPGESLRL SCAASGFTFE DYAMHWVRQP PGKGLEWVSL ISGDGFYTHYADSVEGRFTV SRDNSKNSLY LQMNSLRAED TAVYYCARDS FGGHLDLWGQ GTLVTVSS | 114 |

### Example 4. Identification of Binding Activity of Antibody Variants to COVID-19 Virus-RBD Region

In order to identify the binding activity of six types of scFv antibody variants and four types of VH antibody variants selected in Example 3 above to the COVID-19 virus-RBD region, binding activity analysis was performed using a flow cytometer. To this end, each variant and the Fc (wild type) to be used as a control group were produced from spheroplasts in the same manner as in the above example. The COVID-19 RBD-Alexa 488 antigen used for screening was bound to the produced spheroplasts at a concentration of 50 nM and incubated at room temperature for 1 hour. After washing twice with PBS to remove non-specific binding, the binding activity of antibody variants to the COVID-19 virus-RBD region was analyzed using a flow cytometer. Herein, as a positive control group, CR3022 antibody developed by Crucell (merged with Johnson & Johnson) as a SARS RBD-binding antibody was used (recently known to show excellent binding activity also to SARS-CoV-2 RBD) (References: US Patent US 8,106,176 B2, Tian et al BioRxiv 2020, Joyce et al BioRxiv 2020).

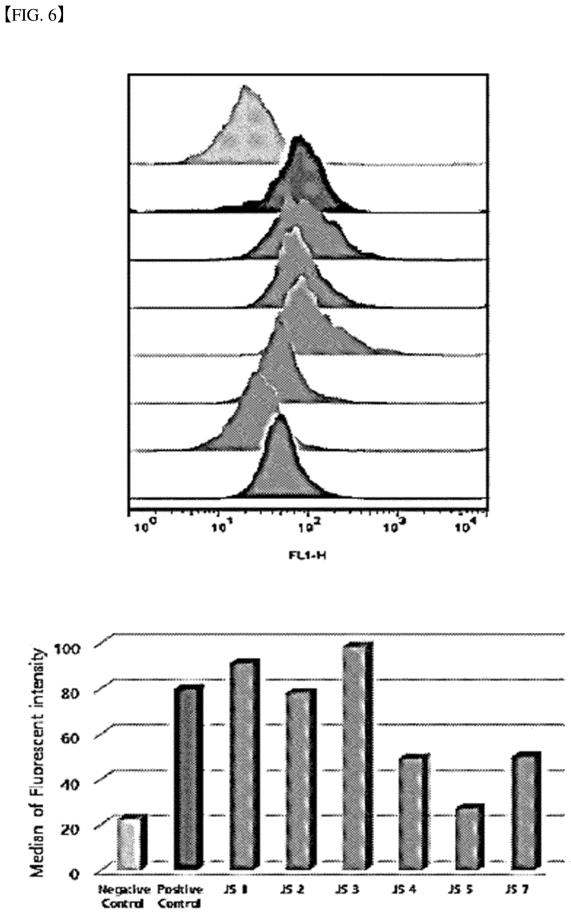

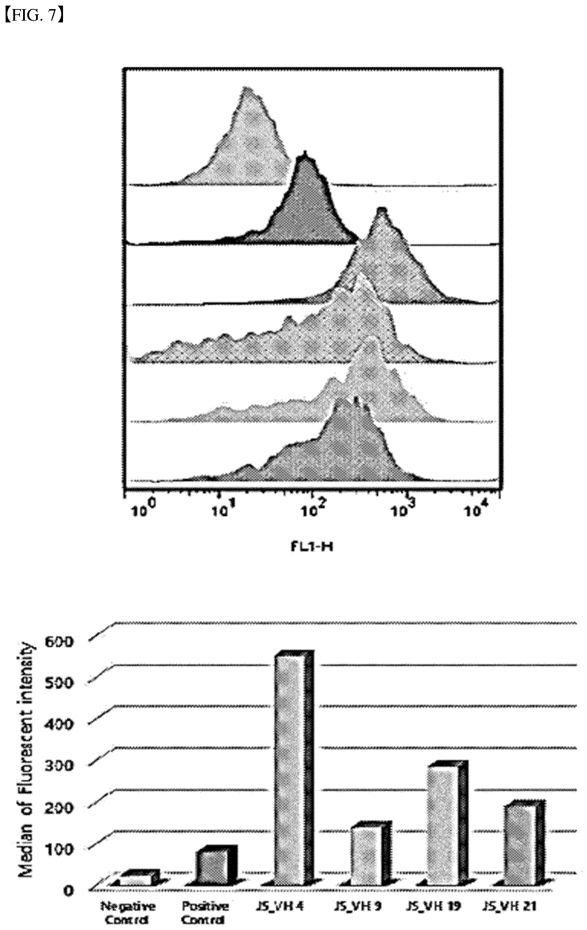

As a result, all six types of scFv variants and four types of VH variants showed significantly increased fluorescence signals compared to the control group of Fc (wild type) (FIGS. 6 and 7). Thereby, it was identified that the scFv antibody variants and VH antibody variants of an embodiment of the present invention had high affinity binding to the COVID-19 virus-RBD region.

### Example 5. Production and Purification of IgG-Type Antibodies of 6 Types of scFv Variants

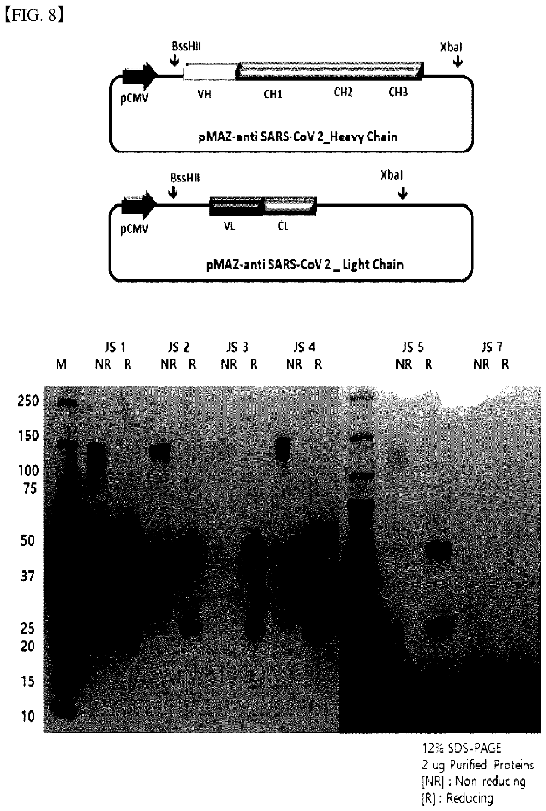

Six types of scFv antibody variants selected in the example above were produced in an IgG form and the binding activity to COVID-19 virus-RBD was identified. To this end, heavy chain and light chain expression vectors of six types of scFv were produced respectively, the heavy chain genes and light chain genes of the variants were mixed in a ratio of 1 : 1, and PEI and variant genes were mixed in a ratio of 4 : 1 and transfected into Expi293F animal cells. Thereafter, the cells were cultured for 7 days under conditions of 37°C, 125 rpm, and 8% CO₂ in a CO₂ stirring incubator, and then centrifuged to collect only a supernatant. The supernatant was equilibrated with 25x PBS and filtered through a 0.2 µm filter (Merck Millipore). The filtered culture solution was added with 500 µl of Protein A resin and stirred at 4°C for 16 hours, and then the resin was recovered through the column. The recovered resin was washed with 5 ml PBS and eluted with 3 ml of 100 mM glycine buffer (pH 2.7), and then neutralized with 1 M Tris-HCl (pH 8.0). The buffer was replaced with PBS using centrifugal filter units 3K (Merck Millipore), and then the size and purity of six types of antibodies purified were analyzed by SDS-PAGE, respectively (FIG. 8).

### Example 6. Identification of Binding Activity of IgG-Type Antibody of scFv Variant to COVID-19 Virus-RBD Region

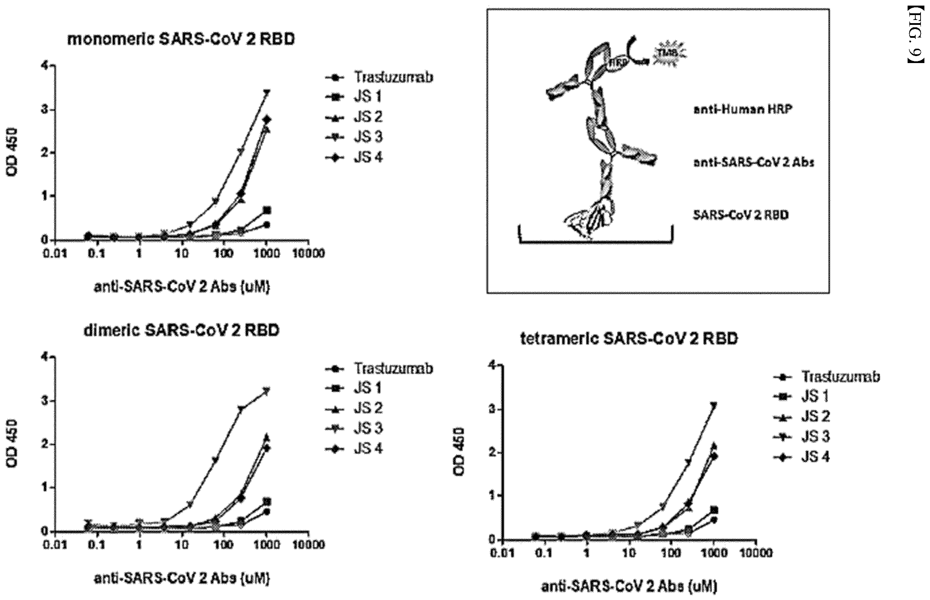

Among the six types of antibodies whose expression and purification were completed in Example 5 above, an ELISA experiment was performed on the antibodies JS 1, JS 2, JS 3, and JS 4. Specifically, 50 µl of dimeric RBD fused with a monomeric RBD and a GST tag or a tetrameric RBD fused with a streptavidin tag was immobilized in different Flat Bottom Polystyrene High Bind 96-well plates (Costar) at 4 µg/ml in 0.05 M Na₂CO₃ pH 9.6 at 4°C for 16 hours, and then was blocked with 100 µl of 4% skim milk (GenomicBase) (in 0.05% PBST pH 6.0/pH 7.4) at room temperature for 2 hours. Thereafter, the above results were washed 4 times with 180 µl of 0.05% PBST, and 50 µl of 4 types of JS 1, JS 2, JS 3, and JS 4 antibodies serially diluted with 1% skim milk (in 0.05% PBST) and Trastuzumab as a control group were dispensed into each well and reacted at room temperature for 1 hour. After the washing process, each antibody was reacted with 50 µl of antihuman HRP conjugate (Jackson Immunoresearch) at room temperature for 1 hour and washed. 1-Step Ultra TMB-ELISA Substrate Solution (Thermo Fisher Scientific) was added at 50 µl each to develop color, and then 2 M H₂SO₄ was added at 50 µl each to terminate the reaction and conduct an analysis using an Epoch Microplate Spectrophotometer (BioTek). As a result, it was identified that the IgG antibodies of JS 1, JS 2, JS 3, and JS 4 all had stronger binding activity to the COVID-19 virus-RBD region than the control group of Trastuzumab (FIG. 9).

### Example 7. Production and Purification of IgG-Type Antibody of VH Variant

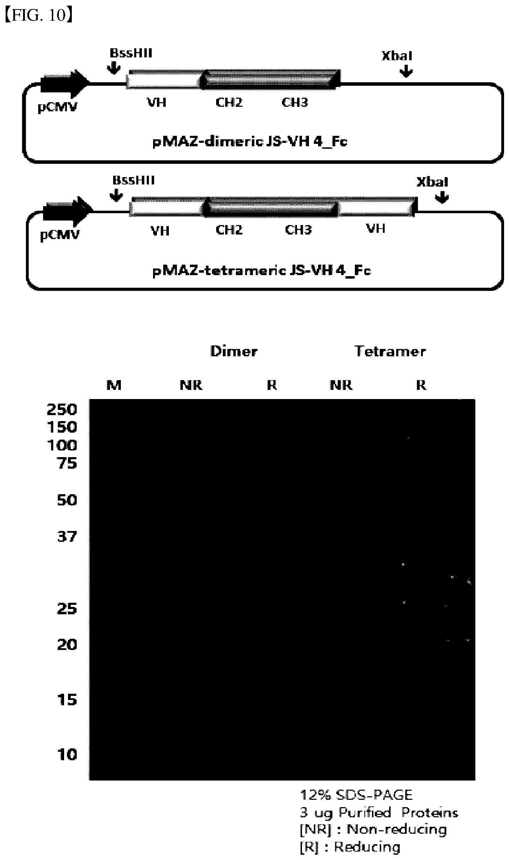

Among four types of VH antibody variants selected in Example 3 above, in order to identify whether the JS-VH 4 variant, which showed the highest binding activity to the COVID-19 virus-RBD as a result of flow cytometric analysis in Example 4, has strong binding activity to COVID-19 virus-RBD on protein even in the form of IgG, the expression vectors in which the JS-VH 4 variants were introduced as dimeric and tetrameric forms into the Fc (Crystallization Fragment), which is the constant region of the antibody, were constructed, respectively, and transfected into Expi293F animal cells. Thereafter, the cells were cultured for 7 days under conditions of 37°C, 125 rpm, and 8% CO₂ in a CO₂ stirring incubator, and then centrifuged to collect only a supernatant. The supernatant was equilibrated with 25x PBS and filtered through a 0.2 µm filter (Merck Millipore). The filtered culture solution was added with 500 µl of Protein A resin and stirred at 4°C for 16 hours, and then the resin was recovered through the column. The recovered resin was washed with 5 ml of PBS and eluted with 3 ml of 100 mM glycine buffer (pH 2.7), and then neutralized with 1 M Tris-HCl (pH 8.0). The buffer was replaced with PBS using centrifugal filter units 3K (Merck Millipore), and then the size and purity of two types of antibodies (dimer and tetramer) purified respectively were analyzed by SDS-PAGE (FIG. 10).

### Example 8. Identification of Binding Activity of IgG-Type Antibody of VH Variant to COVID-19 Virus-RBD Region

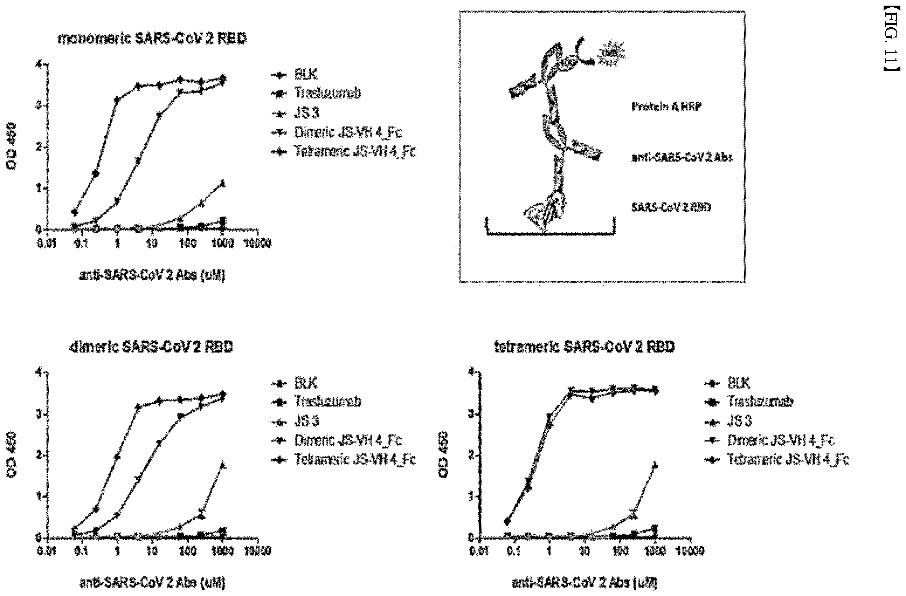

An ELISA experiment was performed on the antibody in the form of IgG of the dimeric or tetrameric JS-VH 4 variant whose expression and purification were completed in Example 7 above. Specifically, 50 µl of dimeric RBD fused with a monomeric RBD and a GST tag or a tetrameric RBD fused with a streptavidin tag was immobilized in different Flat Bottom Polystyrene High Bind 96-well plates (Costar) at 4 µg/ml in 0.05 M Na₂CO₃ pH 9.6 at 4°C for 16 hours, and then was blocked with 100 µl of 4% skim milk (GenomicBase) (in 0.05% PBST pH 6.0/pH 7.4) at room temperature for 2 hours. Thereafter, the above results were washed 4 times with 180 µl of 0.05% PBST, and 50 µl of 2 types of JS-VH 4 antibodies (dimeric JS_VH 4_Fc and tetrameric JS_VH 4_Fc) serially diluted with 1% skim milk (in 0.05% PBST) and Trastuzumab and JS 3 antibody as a control group were dispensed into each well and reacted at room temperature for 1 hour. After the washing process, each antibody was reacted with 50 µl of Protein HRP conjugate (GenScript) at room temperature for 1 hour and washed. 1-Step Ultra TMB-ELISA Substrate Solution (Thermo Fisher Scientific) was added at 50 µl each to develop color, and then 2 M H₂SO₄ was added at 50 µl each to terminate the reaction and conduct an analysis using an Epoch Microplate Spectrophotometer (BioTek). As a result, it was identified that the dimeric JS_VH 4_Fc and tetrameric JS_VH 4_Fc (IgG antibody) all had significantly stronger binding activity to the COVID-19 virus-RBD region than the control group of Trastuzumab. In particular, the binding activity was higher than that of JS 3 (FIG. 11).

### Example 9. Screening of JS-VH 4-Originated Antibody Binding to COVID-19 RBD Region

In order to discover a VL against JS-VH 4 that specifically binds to the COVID-19 RBD and to discover a JS-VH 4-originated antibody with binding activity to the COVID-19 RBD, the present inventors constructed a JS-VH 4-originated VL shuffling library in which JS-VH 4 was collectively introduced into the VH region of the constructed bacterial displayed human scFv antibody library. In addition, screening for human antibodies binding to COVID-19 RBD was performed using the COVID-19-RBD-GST-Alexa488 probe, which is the antigen used to discover the primary antibody, and the VL shuffling library originated from the bacterially displayed JS-VH 4 produced above. Specifically, 1 ml of library cells were cultured in 25 ml of TB medium containing 2% glucose and 40 µg/ml of chloramphenicol at 37°C and 250 rpm for 4 hours, and the cultured cells were cultured in 100 ml of TB medium containing 40 µg/ml of chloramphenicol at a ratio of 1 : 100 to OD600 = 0.5. After cooling at 25°C and 250 rpm for 20 minutes, then adding 1 mM IPTG and induction for 5 hours at 25°C and 250 rpm, and *E. coli* overexpressing the protein was harvested based on OD600 = 8. Thereafter, in order to make the cells into spheroplasts suitable for screening using a flow cytometer, the cells were resuspended using 1 ml of 10 Mm Tris-HCl (pH 8.0), washed twice, and the remaining medium was removed. The outer cell membrane was removed by applying osmotic shock by rotating the cells in 1 ml of STE [0.5 M sucrose, 10 Mm Tris-HCl, 10 Mm EDTA (pH 8.0)] solution at 37°C for 30 minutes. Thereafter, the peptidoglycan layer was removed by rotating a mixed solution of 1 ml of Solution A and 20 µl of 50 mg/ml lysozyme solution for 15 minutes at 37°C, and washed with 1 ml of PBS. 700 µl of PBS and 200 nM (monomer basis) of the COVID-19-RBD-GST-Alexa488 probe were added to 300 µl of the mixed solution and rotated at room temperature for 1 hour to label the spheroplasts with the fluorescent probe. Thereafter, clones showing high fluorescence due to increased antigen binding activity were recovered using a flow cytometer (FIG. 2). The process of selecting spheroplasts showing high affinity to the COVID-19 virus-RBD region was repeated through amplification of scFv genes from recovered clones using a PCR method, culture, expression induction, a spheroplasting process to remove an outer cell membrane and peptidoglycan layer, antigen labeling, and selective gating of flow cytometry (FIG. 3). After the selection process using a flow cytometer and the re-selection process to increase selection purity, the next round of selection process was performed to amplify (enrich) the desired clones after going through an enrichment process, a process of securing selected scFv variant genes through PCR amplification, a subcloning process, and a transformation process. Through this repeated search process of several rounds, five types of scFv variant clones (BM 13, BM 18, BM 23, BM 57, and BM 73) with improved binding activity to the COVID-19 virus-RBD region were secured.

### Example 10. Identification of Binding Activity of ScFv Variants Originated from JS-VH 4 to COVID-19 Virus-RBD Region

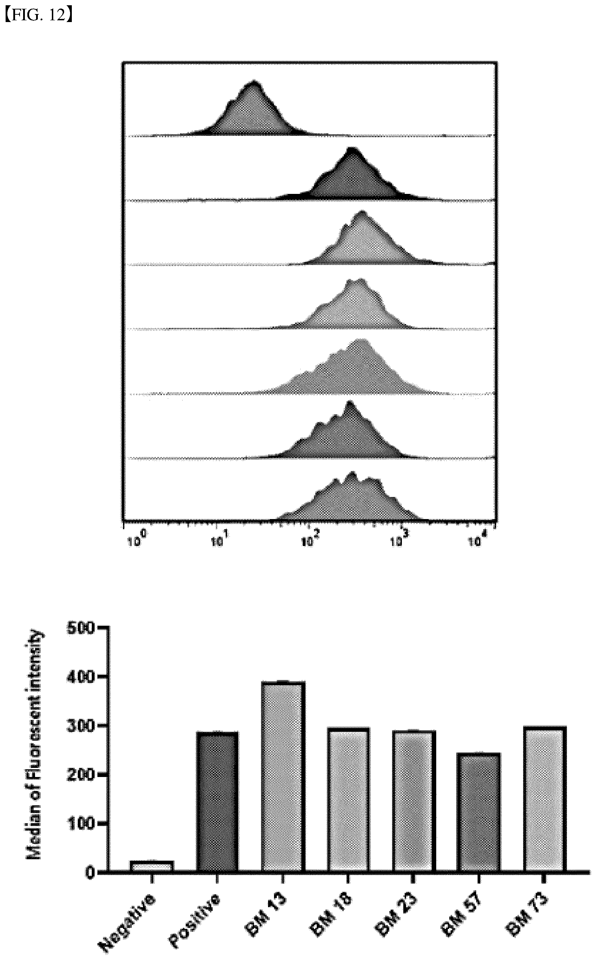

In order to identify the binding activity of the scFv variants originated from JS-VH 4 obtained in Example 9 above to the COVID-19 virus-RBD region, the binding activity analysis was performed using a flow cytometer. To this end, each variant and JS-VH 4 to be used as a control group were produced into spheroplasts, respectively, in the same manner as in the above example. The COVID-19 RBD-Alexa 488 antigen used for screening was bound to the produced spheroplasts at a concentration of 25 nM and incubated at room temperature for 1 hour. After washing twice with PBS to remove non-specific binding, the binding activity of each scFv variant to the COVID-19 virus-RBD region was analyzed using a flow cytometer. As a result of the analysis, it was identified that five types of scFv variants showed fluorescence signals similar to JS-VH 4 (FIG. 12).

### Example 11. Identification of Gene Sequences of scFv Variants Originated from JS-VH 4

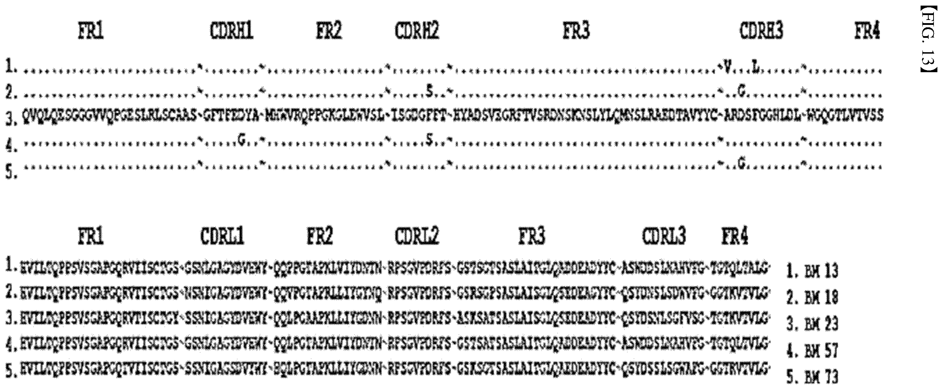

In order to identify the gene sequences of five types of scFv variant clones obtained in Example 9 above, the DNA base sequences were analyzed using Sanger sequencing. Among five types of variants, four types of scFv variants excluding BM 23 were screened using a VL shuffling library originated from JS-VH 4, in which JS-VH 4 was collectively introduced. However, it was identified that amino acid substitutions also occurred in the VH region during the PCR amplification process (FIG. 13). In addition, the VH amino acid sequences of four types of scFv variants originated from JS-VH 4 (BM 13, BM 18, BM 57 and BM 73) and the VL amino acid sequences of five types of scFv variants originated from JS-VH 4 (BM 13, BM 18, BM23, BM 57, and BM 73) were identified (Table 2).

**[Table 2]**

| **Variants** | **Regions** | **Sequences** | **SEQ ID NO** |
|---|---|---|---|
| BM 13 | VH | QVQLQESGGG VVQPGESLRL SCAASGFTFE DYAMHWVRQP PGKGLEWVSL ISGDGFFTHYADSVEGRFTV SRDNSKNSLY LQMNSLRAED TAVYYCVRDS LGGHLDLWGQ GTLVTVSS | 115 |
| | V_ | HVILTQPPSV SGAPGQRVII SCTGSGSNLG AGYDVHWYQQ PPGTAPKLVI YDNTNRPSGVPDRFSGSTSG TSASLAITGL QADDEADYYC ASWDDSLNAH VFGTGTQLTA LG | 127 |
| BM 18 | VH | QVQLQESGGG VVQPGESLRL SCAASGFTFE DYAMHWVRQP PGKGLEWVSL ISGDGSFTHY ADSVEGRFTV SRDNSKNSLY LQMNSLRAED TAVYYCARGS FGGHLDLWGQ GTLVTVSS | 116 |
| | V_ | HVILTQPPSV SGAPGQRVTI SCTGSNSNIG AGYDVHWYQQ VPGTAPRLLI YGYNQRPSGVPDRFSGSRSG PSASLAISGL QSEDEAGYYC QSYDNSLSDW VFGGGTKVTV LG | 123 |
| BM 57 | VH | QVQLQESGGG VVQPGESLRL SCAASGFTFE GYAMHWVRQP PGKGLEWVSL ISGDGSFTHYADSVEGRFTV SRDNSKNSLY LQMNSLRAED TAVYYCARDS FGGHLDLWGQ GTLVTVSS | 117 |
| | V_ | HVILTQPPSV SGAPGQRVII SCTGSGSNLG AGYDVHWYQQ LPGTAPKLVI YDNTNRPSGVPDRFSGSTSA TSASLAITGL QADDEADYYC ASWDDSLNAH VFGTGTQLTV LG | 129 |
| BM 73 | VH | QVQLQESGGG VVQPGESLRL SCAASGFTFE DYAMHWVRQP PGKGLEWVSL ISGDGFFTHYADSVEGRFTV SRDNSKNSLY LQMNSLRAED TAVYYCARGS FGGHLDLWGQ GTLVTVSS | 119 |
| | V_ | HVILTQPPSV SGAPGQTVII SCTGSSSNIG AGSDVYWYHQ LPGTAPKLLI YGDNNRPSGVPDRFSGSKSG TSASLAITGL QAEDEADYYC QSYDSSLSGW AFGGGTRVTV LG | 130 |
| BM 23 | VH | QVQLQESGGG VVQPGESLRL SCAASGFTFE DYAMHWVRQP PGKGLEWVSL ISGDGFFTHY ADSVEGRFTV SRDNSKNSLY LQMNSLRAED TAVYYCARDS FGGHLDLWGQ GTLVTVSS | 119 |
| | V_ | HVILTQPPSV SGAPGQTVII SCTGSSSNIG AGSDVYWYHQ LPGTAPKLLI YGDNNRPSGVPDRFSGSKSG TSASLAITGL QAEDEADYYC QSYDSSLSGW AFGGGTRVTV LG | 131 |
| S18.1 | VH | QVQLQESGGG VVQPGESLRL SCAASGFTFE DYDMHWVRQP LGKGLEWVSL ISGDGSFTHY ADSVEGRFTV SRDNSKNSLY LQMNSLRAED TAVYYCARGS FGGLLDLWGQ GTLVTVSS | 120 |
| | V_ | HVILTQPPSV SGTPGQRVTI SCTGSNSNIG AGYDVHWYQQ VPGTAPRLLI YGYNQRPSGVPDRFSGSRSG PSASLAISGL QSEDEAGYYC QSYDYSLSDW VFGGGTEVTV LG | 132 |

### Example 12. Identification of Increase in COVID-19 Virus-RBD Region Binding Activity by Substitution of VH Region Amino Acids

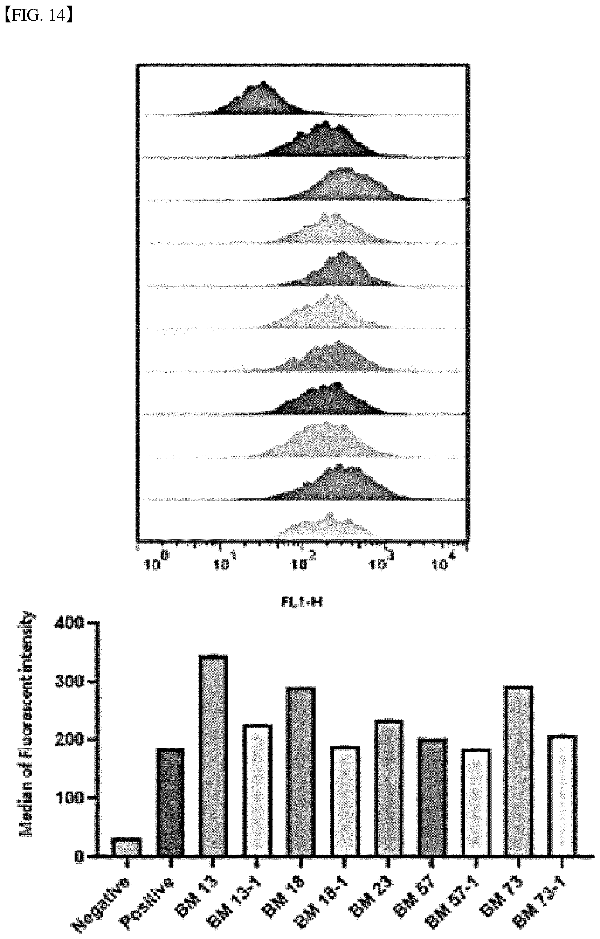

As a result of checking the sequences in Example 11 above, the amino acid substitution mutations of four types of scFv variants (BM 13, BM 18, BM 57, and BM 73) with amino acid substitutions in the VH region were substituted with JS-VH 4 again to identify the effect of the newly introduced amino acid on binding to the COVID-19 virus-RBD in the VH region. As a result, when all four types of variants were substituted with JS-VH 4 again, the binding to the COVID-19 virus-RBD was found to be reduced (FIG. 14), thus identifying that the newly discovered amino acids in the VH region improved the binding activity to the COVID-19 virus-RBD.

### Example 13. Production of RBD (Receptor Binding Domain) Antigen of SARS-CoV-2 Virus Delta Variant

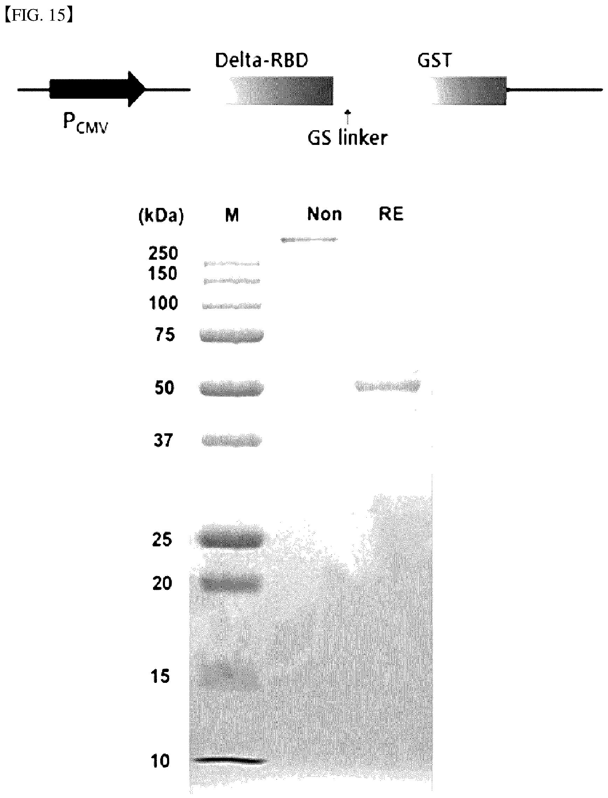

An antigen was produced to induce directed evolution of antibodies against the Delta (B.1.617.2) variant, which is known to be the most transmissible among various variants of the COVID-19 virus. Specifically, in order to create an antibody that binds to the RBD (receptor binding domain) region, which is known to be important for the virus to infect the host by binding to the surface of the ACE2 (angiotensin converting enzyme 2) receptor present on host cells, the RBD region of the SARS-CoV-2 virus delta variant was prepared as an antigen. In addition, in order to produce an antigen more suitable for screening an antibody using a flow cytometer, vectors for animal cell expression were constructed to produce antigens in a dimeric form fused with a GST tag to the antigen. After mixing 30 ml of Freestyle 293 expression culture solution (Gibco, 12338-018) with each vector, a solution of PEI (polyethylenimine) (Polyscience, 23966) and each vector mixed at a 4 : 1 ratio was left at room temperature for 20 minutes and then treated and transfected into 300 ml of Expi293F animal cells subcultured the previous day at a density of 2 × 10⁶ cells/ml. Thereafter, the transfected cells were incubated for 7 days under conditions of 37°C, 125 rpm, and 8 % CO₂ in a shaking CO₂ incubator, and then centrifuged to collect only a supernatant. Thereafter, the supernatant was equilibrated with 25x PBS and filtered through a 0.2 µm filter (Merck Millipore) using a bottle top filter. The filtered culture solution was added with 1 ml of GSH resin and stirred at 4°C for 16 hours, and then the resin was recovered through the column, washed with 5 ml of PBS and eluted with 4 ml of 50 mM Tris-HCl & 10 mM reduced glutathione (pH 8.0) (SIGMA). The buffer was replaced with PBS, and then the size and purity of the purified antigen protein SARS-CoV-2 virus Delta-RBD-GST were analyzed, respectively, under non-reducing conditions (Non) and reducing conditions (RE) through SDS-PAGE (FIG. 15). In addition, the SARS-CoV-2 virus Delta-RBD-GST was labeled with the Alexa488 fluorescent molecule for use in screening using a flow cytometer.

### Example 14. Production of Library for Screening Antibodies with Improved Binding Activity to RBD of SARS-CoV-2 Virus Delta Variants

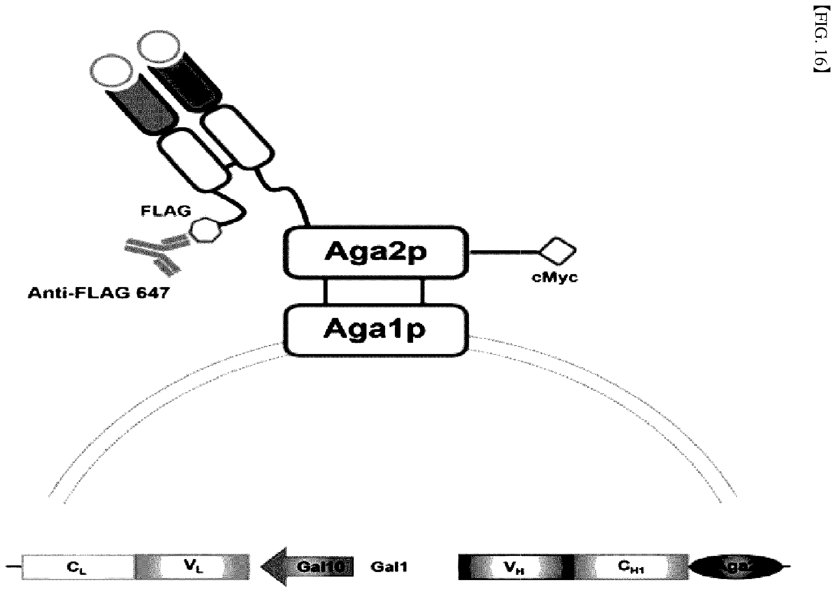

In order to efficiently select human antibodies with improved binding activity to the SARS-CoV-2 virus Delta variant, a library containing random mutations at 0.2% was produced through error prone PCR in the variable regions of BM 18 and BM 23 (scFv variant originated from JS-VH 4 which binds to the wild-type COVID-19 virus-RBD region), which are displayed in Fab form in yeast. The produced DNA library was delivered to 400 µl of yeast *Saccharomyces cerevisiae* competent cells through electroporation using a MicroPulser Electroporator (Bio-Rad) along with 8 µg of a vector encoding the yeast cell wall anchoring protein, which is Aga2 protein and the transformed selection marker gene (Trp1). Through the process of culturing in 500 ml of SD medium [Difco Yeast nitrogen base (BD) 6.7 g/l, Bacto casamino acid (BD) 5.0 g/l, Na₂HPO₄ (JUNSEI) 5.4 g/l, NaH₂PO₄.H₂O (SAMCHUN) 8.56 g/l and Glucose (DUKSAN) 20 g/l], only the transformed yeast species were selectively allowed to survive, thereby producing yeast display libraries with 7.5 × 10⁷ and 1.0 × 10⁸ diversities for BM 18 and BM 23, respectively (FIG. 16).

### Example 15. Screening of Human Antibodies with Improved Binding Activity to COVID-19 Virus Delta Variants

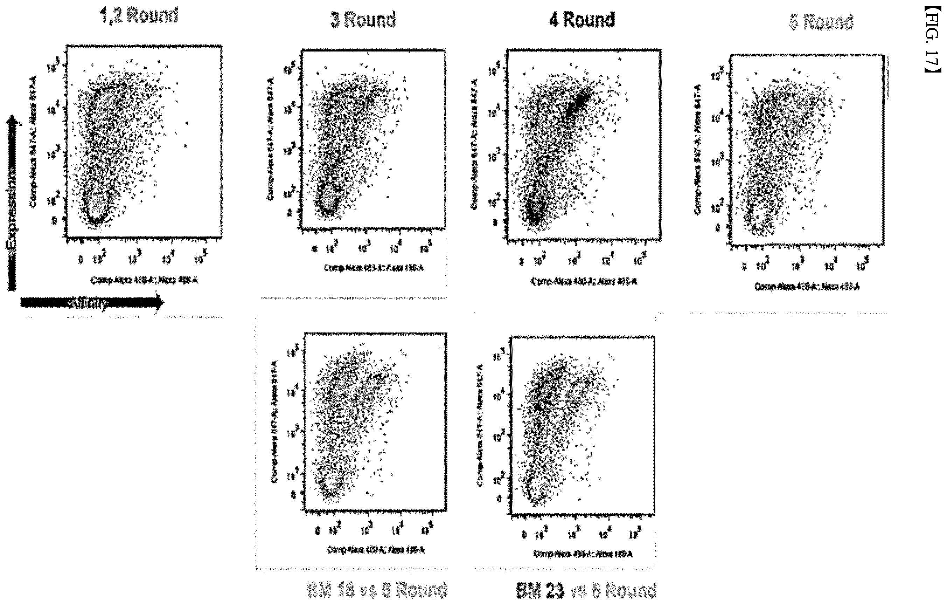

In order to discover human antibodies that bind to various COVID-19 variants, a yeast-displayed Fab antibody library was used to screen human antibodies binding to the RBD region of the dimeric COVID-19 virus delta variant (SARS-CoV-2 virus Delta) labeled with the Alexa488 fluorescent molecule using a flow cytometer. Specifically, 5.5 × 10⁸ cells of the yeast library produced in Example 2 above were cultured in 100 ml of tryptophan-deficient SD medium at 30°C for 16 hours, and then 7 × 10⁸ cells were cultured in 100 ml of SG medium [Difco Yeast nitrogen base (BD) 6.7 g/l, Bacto casamino acid (BD) 5.0 g/l, Na₂HPO₄ (JUNSEI) 5.4 g/l, NaH₂PO₄.H₂O (SAMCHUN) 8.56 g/l, Galactose (Sigma-Aldrich) 20 g/l] at 20°C for 48 hours to improve the expression level of the library. Among the cultured cells, 1 × 10⁸ cells were centrifuged at 14,000×g for 30 seconds at 4°C, the supernatant was removed, and washed with 1 ml PBSB (1×PBS, 0.1% bovine serum albumin (gibco) (pH 7.4). Then, 200 nM Alexa488-conjugated SARS-CoV2 delta RBD and anti-FLAG-iFlour (GenScript) of 1000 : 1 produced in Example 1 above were incubated in 1 ml of PBSB (pH 7.4) diluted for 1 hour. After incubation, the cells were washed with 1 ml PBSB (pH 7.4) and resuspended in 1 ml PBSB (pH 7.4) to induce binding to Alexa488-conjugated SARS-CoV2 delta RBD. Cells showing high fluorescence signals were then recovered using a flow cytometer (Bio-Rad S3 sorter, Bio-Rad). For the recovered sub-library, the concentration of Alexa488-conjugated SARS-CoV2 delta RBD was reduced in the same way, and cells displaying Fab variants with excellent binding activity to SARS-CoV2 delta RBD were concentrated through a total of 5 rounds of screening processes (FIG. 17), and 50 individual clone antibodies were obtained.

### Example 16. Identification of Gene Sequence of Antibody Variant Binding to COVID-19 Virus Delta Variant

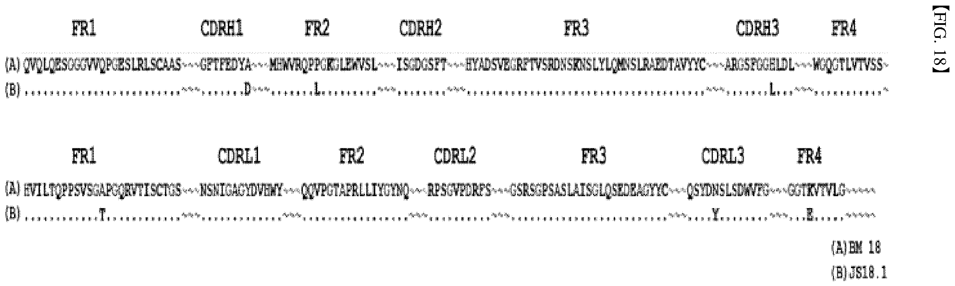

In order to identify the gene sequences of the scFv variant clones obtained in Example 15 above, the DNA base sequences were analyzed using Sanger sequencing. As a result, it was identified that all 50 individual clone antibodies requested for analysis were Fab antibodies with one sequence, and that they were originated from BM 18, the parent antibody, and were named JS18.1 (FIG. 18 and Table 2).

### Example 17. Antibody Production and Purification of COVID-19 Virus Delta Variant Binding Fab Variant

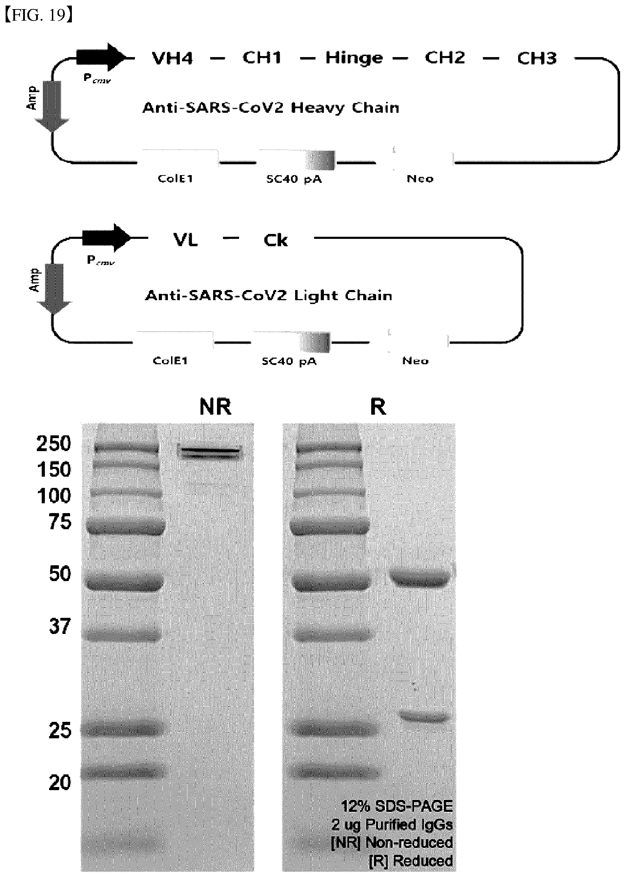

In order to identify whether the Fab antibody variant JS18.1 selected in the above example has binding activity to the RBD of the COVID-19 virus delta variant even in the form of IgG, heavy chain and light chain expression vectors were produced respectively, the heavy chain genes and light chain genes of the variants were mixed in a ratio of 1 : 1, and PEI and variant genes were mixed in a ratio of 4 : 1 and transfected using a PEI (Polyethylenimine, Polyscience, 23966) into Expi293F animal cells which were left at room temperature for 20 minutes and subcultured the previous day at a density of 2 × 10⁶ cells/ml. Thereafter, the cells were cultured for 7 days under conditions of 37°C, 125 rpm, and 8% CO₂ in a CO₂ stirring incubator, and then centrifuged to collect only a supernatant. The supernatant was equilibrated with 25x PBS and filtered through a 0.2 µm filter (Merck Millipore) using a bottle top filter. The filtered culture solution was added with 500 µl of Protein A resin and stirred at 4°C for 16 hours, and then the resin was recovered through the column. The recovered resin was washed with 5 ml of PBS and eluted with 3 ml of 100 mM glycine buffer (pH 2.7), and then neutralized with 1 M Tris-HCl (pH 8.0). The buffer was replaced with PBS using centrifugal filter units 3K (Merck Millipore), and then the size and purity of the antibodies purified were analyzed by SDS-PAGE (FIG. 19).

### Example 18. Analysis of Binding Activity with Various COVID-19 Virus Variants

### 18-1. Production of Monomeric RBD Proteins of Various COVID-19 Virus Variants

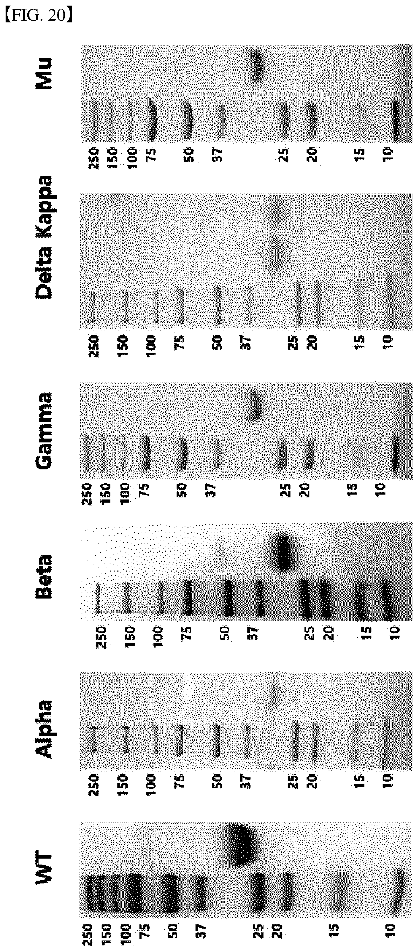

In order to identify the binding activity of the novel antibody JS18.1 discovered through the above example to various variants of COVID-19 virus, the monomeric form of the RBD proteins of a COVID-19 virus (SARS-CoV-2) wild type, a COVID-19 virus alpha variant, a COVID-19 virus beta variant, a COVID-19 virus gamma variant, a COVID-19 virus delta variant, a COVID-19 virus kappa variant, and a COVID-19 virus mu variant were expressed. Specifically, vectors for expression in animal cells were produced to express proteins fused with a polyhistidine tag. After mixing 30 ml of Freestyle 293 expression culture solution (Gibco, 12338-018) with each vector, a solution of PEI (polyethylenimine) (Polyscience, 23966) and RBD variant gene mixed at a 4 : 1 ratio was left at room temperature for 20 minutes and then treated and transfected into Expi293F animal cells subcultured the previous day at a density of 2 × 10⁶ cells/ml. Thereafter, the transfected cells were incubated for 7 days under conditions of 37°C, 125 rpm, and 8% CO₂ in a shaking CO₂ incubator, and then centrifuged to collect only a supernatant. Thereafter, the supernatant was equilibrated with 25x PBS and filtered through a 0.2 µm filter (Merck Millipore) using a bottle top filter. The filtered culture solution was added with 1 ml of Ni-NTA resin and stirred at 4°C for 16 hours, and then the resin was recovered through the column, washed with 5 ml of PBS and eluted with 250 mM imidazole (pH 8.0). The buffer was replaced with PBS using Centrifugal filter units 3K (Merck Millipore), and then the size and purity of the purified antigen proteins were analyzed through SDS-PAGE (FIG. 20).

### 18-2. Binding Activity Analysis for Various COVID-19 Virus Variants

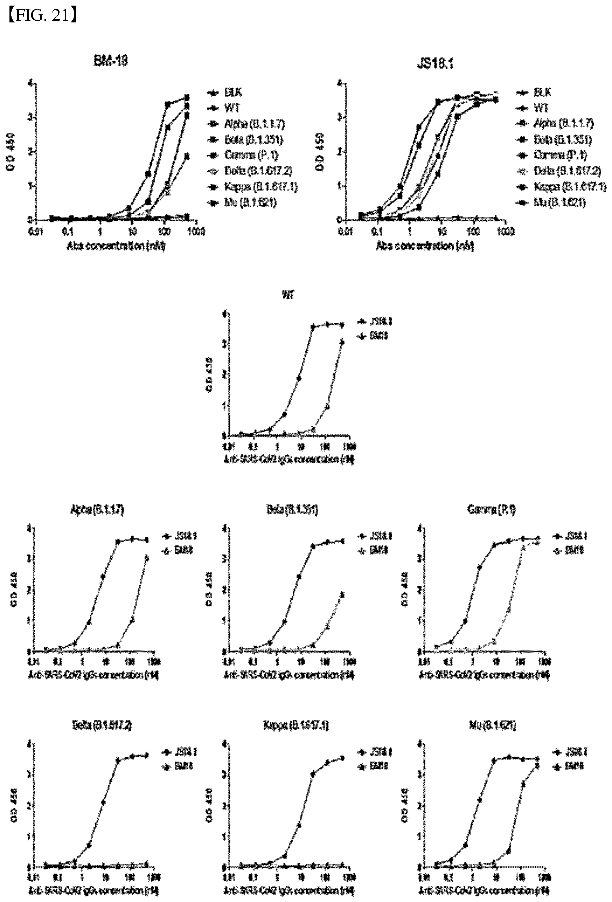

In order to analyze the antibody binding activity to the RBD of various COVID-19 virus variants of JS18.1, a Fab variant that binds to the COVID-19 virus delta variant derived from the above example, the antigen proteins produced in 6-1 above were used to perform an ELISA experiment on the JS18.1 antibody whose expression and purification were completed in the above example and its parent antibody, BM 18. Specifically, 50 µl each of the monomeric RBD proteins of a COVID-19 virus (SARS-CoV-2) wild type, a COVID-19 virus alpha variant, a COVID-19 virus beta variant, a COVID-19 virus gamma variant, a COVID-19 virus delta variant, a COVID-19 virus kappa variant, and a COVID-19 virus mu variant of Example 6-1 above, which were diluted to be 4 µg/ml in 0.05 M Na₂CO₃ pH 9.6, was incubated and immobilized in different Flat Bottom Polystyrene High Bind 96-well plates (Costar) at 4°C for 16 hours, and then was blocked with 100 µl of 4% skim milk (GenomicBase) and 0.05% PBST (pH 7.4) at room temperature for 2 hours. Thereafter, the above results were washed 4 times with 180 µl of 0.05% PBST, and 50 µl each of the JS18.1 antibody and the BM 18 antibody serially diluted with 1% skim milk (in 0.05% PBST) was dispensed into each well and reacted at room temperature for 1 hour. After the washing process, each antibody was reacted with 50 µl of anti-human HRP conjugate (Jackson Immunoresearch) at room temperature for 1 hour and washed. Thereafter, 1-Step Ultra TMB-ELISA Substrate Solution (Thermo Fisher Scientific) was added at 50 µl each to develop color, and then 2 M H₂SO₄ was added at 50 µl each to terminate the reaction and analyze absorbance using an Epoch microplate spectrophotometer (BioTek). As a result, it was identified that the JS18.1 antibody of an embodiment of the present invention was bound to all of a COVID-19 virus wild type, a COVID-19 virus alpha variant, a COVID-19 virus beta variant, a COVID-19 virus gamma variant, a COVID-19 virus delta variant, a COVID-19 virus kappa variant, and a COVID-19 virus mu variant with high affinity, and had significantly increased binding characteristics compared to its parent antibody, BM 18 (FIG. 21).

## Claims

1. An antibody specific to COVID-19 virus (SARS-CoV-2) or a variant thereof, or an immunologically active fragment thereof.

2. The antibody or the immunologically active fragment thereof of claim 1, wherein the variant is an alpha variant, a beta variant, a gamma variant, a delta variant, a kappa variant, or a mu variant.

3. The antibody or the immunologically active fragment thereof of claim 1, wherein the immunologically active fragment is any one selected from the group consisting of Fab, Fd, Fab', dAb, F(ab'), F(ab')₂, single chain fragment variable (scFv), Fv, single chain antibody, Fv dimer, complementarity determining region fragment, humanized antibody, a chimeric antibody, and a diabody.

4. The antibody or the immunologically active fragment thereof of claim 1, wherein the antibody specific to COVID-19 virus (SARS-CoV-2) or the variant thereof, or the immunologically active fragment thereof comprises a VH domain comprising complementarity determining regions heavy chain (CDRH)1, which comprises any one selected from the group consisting of amino acid sequences represented by SEQ ID NOs: 1 to 8, CDRH2, which comprises any one selected from the group consisting of amino acid sequences represented by SEQ ID NOs: 9 to 14, and CDRH3, which comprises any one selected from the group consisting of amino acid sequences represented by SEQ ID NOs: 15 to 23.

5. The antibody or the immunologically active fragment thereof of claim 1, wherein the antibody specific to COVID-19 virus (SARS-CoV-2) or the variant thereof, or the immunologically active fragment thereof comprises a VH domain comprising FR1, which comprises any one selected from the group consisting of amino acid sequences represented by SEQ ID NOs: 47 to 50, FR2, which comprises any one selected from the group consisting of amino acid sequences represented by SEQ ID NOs: 51 to 56, FR3, which comprises any one selected from the group consisting of amino acid sequences represented by SEQ ID NOs: 57 to 61, and FR4, which comprises an amino acid sequence represented by SEQ ID NO: 62 or 65.

6. The antibody or the immunologically active fragment thereof of claim 1, wherein the antibody specific to COVID-19 virus (SARS-CoV-2) or the variant thereof, or the immunologically active fragment thereof comprises a VL domain comprising complementarity determining regions light chain (CDRL)1, which comprises any one selected from the group consisting of amino acid sequences represented by SEQ ID NOs: 24 to 32, CDRL2, which comprises any one selected from the group consisting of amino acid sequences represented by SEQ ID NOs: 33 to 37, and CDRL3, which comprises any one selected from the group consisting of amino acid sequences represented by SEQ ID NOs: 38 to 46.

7. The antibody or the immunologically active fragment thereof of claim 1, wherein the antibody specific to COVID-19 virus (SARS-CoV-2) or the variant thereof, or the immunologically active fragment thereof comprises a VL domain comprising FR1, which comprises any one selected from the group consisting of amino acid sequences represented by SEQ ID NOs: 66 to 75, FR2, which comprises any one selected from the group consisting of amino acid sequences represented by SEQ ID NOs: 76 to 85, FR3, which comprises any one selected from the group consisting of amino acid sequences represented by SEQ ID NOs: 86 to 94, and FR4, which comprises any one selected from the group consisting of amino acid sequences represented by SEQ ID NO: 95 to 103.

8. The antibody or the immunologically active fragment thereof of claim 1, wherein the antibody specific to COVID-19 virus (SARS-CoV-2) or the variant thereof, or the immunologically active fragment thereof comprises a V domain comprising (i) a VH domain comprising CDRH1, which comprises any one selected from the group consisting of amino acid sequences represented by SEQ ID NOs: 1 to 8, CDRH2, which comprises any one selected from the group consisting of amino acid sequences represented by SEQ ID NOs: 9 to 14, and CDRH3, which comprises any one selected from the group consisting of amino acid sequences represented by SEQ ID NOs: 15 to 23; and/or (ii) a VL domain comprising CDRL1, which comprises any one selected from the group consisting of amino acid sequences represented by SEQ ID NOs: 24 to 32, CDRL2, which comprises any one selected from the group consisting of amino acid sequences represented by SEQ ID NOs: 33 to 37, and CDRL3, which comprises any one selected from the group consisting of amino acid sequences represented by SEQ ID NOs: 38 to 46.

9. The antibody or the immunologically active fragment thereof of claim 1, wherein the antibody specific to COVID-19 virus (SARS-CoV-2) or the variant thereof, or the immunologically active fragment thereof comprises a VH domain, which comprises any one selected from the group consisting of amino acid sequences represented by SEQ ID NOs: 104 to 119; and a VL domain, which comprises any one selected from the group consisting of amino acid sequences represented by SEQ ID NOs: 120 to 131.

10. An isolated nucleic acid molecule encoding the antibody or the immunologically active fragment thereof of claim 1.

11. A vector comprising the isolated nucleic acid molecule of claim 10.

12. A host cell transformed with the vector of claim 11.

13. A method of preparing an antibody specific to COVID-19 virus or a variant thereof or an immunologically active fragment thereof, the method comprising:
a) culturing a host cell comprising a vector comprising the isolated nucleic acid molecule; and
b) recovering the antibody or the immunologically active fragment thereof from host cell culture.

14. A pharmaceutical composition for preventing or treating COVID-19 virus infection, the composition comprising the antibody specific to COVID-19 virus or the variant thereof, or the immunologically active fragment thereof of claim 1.

15. The pharmaceutical composition of claim 14, wherein the COVID-19 virus infection is caused by infection with a COVID-19 virus (SARS-CoV-2) wild type, a COVID-19 virus alpha variant, a COVID-19 virus beta variant, a COVID-19 virus gamma variant, a COVID-19 virus delta variant, a COVID-19 virus kappa variant, or a COVID-19 virus mu variant.

16. A composition for detecting COVID-19 virus or a variant thereof, the composition comprising the antibody specific to COVID-19 virus or the variant thereof, or the immunologically active fragment thereof of claim 1.

17. A kit for detecting COVID-19 virus or a variant thereof, the kit comprising the composition of claim 16.

18. A method for detecting COVID-19 virus or a variant thereof, the method comprising:
(a) contacting a sample separated from a specimen with the antibody specific to the COVID-19 virus or the variant thereof or the immunologically active fragment thereof of claim 1; and
(b) detecting a formation of an antigen-antibody complex.

19. The method of claim 18, wherein the variant is an alpha variant, a beta variant, a gamma variant, a delta variant, a kappa variant, or a mu variant.

20. A method of providing information regarding diagnosis of COVID-19 virus infection, the method comprising:
(a) contacting a sample separated from a specimen with the antibody specific to the COVID-19 virus or the variant thereof or the immunologically active fragment thereof of claim 1 to form an antigen-antibody complex; and
(b) detecting a formation of the complex.

21. The method of claim 20, wherein the COVID-19 virus infection is caused by infection with a COVID-19 virus (SARS-CoV-2) wild type, a COVID-19 virus alpha variant, a COVID-19 virus beta variant, a COVID-19 virus gamma variant, a COVID-19 virus delta variant, a COVID-19 virus kappa variant, or a COVID-19 virus mu variant.

22. A use of an antibody specific to COVID-19 virus (SARS-CoV-2) or a variant thereof, or an immunologically active fragment thereof, for use in preparing a therapeutic agent against COVID-19 virus infection.

23. A use of an antibody specific to COVID-19 virus (SARS-CoV-2) or a variant thereof, or an immunologically active fragment thereof, for preventing or treating COVID-19 virus infection.

24. A method of treating COVID-19 virus infection, the method comprising administering a pharmaceutically effective amount of an antibody specific to COVID-19 virus (SARS-CoV-2) or a variant thereof, or an immunologically active fragment thereof, to a subject suffering from the COVID-19 virus infection.
